# EUROPEAN PATENT APPLICATION

(11) **EP 4 461 721 A1**
(43) Date of publication of application: **13.11.2024**
(21) Application number: 22918609.3
(22) Date of filing: 06.01.2022
(51) Int. Cl.: C07C 35/37, B01D 53/14, B01J 20/22, B01J 20/24, B32B 27/00, B32B 27/18, C07C 235/02, C07C 265/10, C08G 18/28, C08G 18/32, C09D 7/63, C09D 201/00, C09J 11/06, C09J 175/04, C09J 201/00

(54) **OXYGEN-ABSORBING COMPOUND, AND OXYGEN-ABSORBING ADHESIVE COMPOSITION AND OXYGEN-ABSORBING COATING AGENT COMPOSITION EACH USING SAME**

(71) Applicant: Dai Nippon Printing Co., Ltd., Tokyo 162-8001 (JP); Rock Paint Co., Ltd., Osaka-shi, Osaka 555-0033 (JP)
(72) Inventor: UCHIDA, Nozomu, Tokyo 136-0076 (JP); NUMATA, Mutsuaki, Tokyo 136-0076 (JP); NIHEI, Eita, Tokyo 136-0076 (JP); SAITO, Fumihiko, Tokyo 162-8001 (JP); OIKAWA, Wataru, Tokyo 162-8001 (JP); KATSUMATA, Shiori, Tokyo 162-8001 (JP); HAMANO, Mie, Tokyo 162-8001 (JP)
(74) Representative: Müller-Boré & Partner Patentanwälte PartG mbB
(86) International application number: PCT/JP2022/000154
(87) International publication number: WO 2023/132020

(57) **Abstract**

An object of the present invention is to provide: an oxygen-absorbing compound which has oxygen-absorbing properties and generates less odor, and an oxygen-absorbing adhesive composition and a coating agent composition each produced using the oxygen-absorbing compound. The oxygen-absorbing adhesive composition is used as an adhesive composition for forming an adhesive layer in a laminate for a packaging material, generates less odor, absorbs oxygen in a packaging bag or a packaging container to prevent the deterioration of a content in the packaging bag or the packaging container, and has an excellent balance among adhesion strength, the amount of generated odor and oxygen adsorption properties. The oxygen-absorbing coating agent composition is used as a coating agent composition for forming a coating layer for the surface of the laminate for a packaging material, generates less odor, absorbs oxygen penetrating through the laminate or oxygen in a packaging bag or a packaging container to prevent the deterioration of the coating layer or another constituent layer in the laminate or the deterioration of a content in the packaging bag or the packaging container due to the oxygen, and has an excellent balance among blocking resistance, the amount of generated odor and oxygen absorbing properties. The oxygen-absorbing compound has one or two or more unsaturated five-membered rings, wherein any bond between five carbon atoms constituting the unsaturated five-membered ring is a carbon-carbon double bond, when there is one unsaturated five-membered ring, the five-membered ring or the organic group 1 has a functional group having active hydrogen or a group in which active hydrogen of the functional group having active hydrogen is replaced with a monovalent organic group 2, and when there are two or more unsaturated five-membered rings, the unsaturated five-membered rings are bonded to each other via a divalent or higher organic group 2 that replaces active hydrogen of an active hydrogen group on each of the five-membered rings or the organic group 1.

## Description

### Technical Field

The present invention relates to an oxygen-absorbing compound to be mixed into a resin composition and absorb oxygen in the air, an oxygen-absorbing adhesive composition produced with the oxygen-absorbing compound, and a coating agent composition produced with the oxygen-absorbing adhesive composition.

The oxygen-absorbing adhesive composition is used as an adhesive composition for forming an adhesive layer in a laminate for packaging materials, and is an oxygen-absorbing adhesive composition that absorbs oxygen in a packaging bag or packaging container.

The coating agent composition is used as a coating agent for forming a coating layer on the surface of a laminate for packaging materials, and is a coating agent composition that absorbs oxygen permeating the laminate or oxygen inside a packaging bag or packaging container.

Examples of the use of the coating agent composition include coating to films used in package, or application for the inner surfaces of bottles or the inner surfaces of food wrap films.

### Background Art

As conventional packaging methods to prevent the quality in the contents of food, medical products, chemical products, cosmetics, detergents, metal parts, electronic parts, and the like from deteriorating due to oxygen, packaging materials with high oxygen barrier properties are used, gas replacement is performed with inert gas such as nitrogen gas in the contents-accommodating portion, or oxygen absorbers are packaged with reduced iron powder, which causes problems such as insufficient performance, increased packaging costs, increased waste, only functioning in moist environments, and the risk of accidental ingestion.

Further, in Patent Literature 1, a packaging material with a resin that has oxygen-absorbing properties and generates less odor is also developed, but this resin is polycyclododecene that is insoluble in polar solvents and does not have active hydrogen groups, and its application is limited.

In Patent Literature 2, an adhesive agent for lamination made of a resin with methyltetrahydrophthalic acid as a raw material having oxygen-absorbing properties is proposed, but has the drawback of being unstable due to low oxygen-absorbing properties.

Patent Literature 3 describes oxygen-absorbing thermoplastic resin having a repeating unit consisting of a saturated five-membered ring having a substituent containing a carbon-carbon double bond and a -CH=CH-group connecting the saturated five-membered rings, and there are disadvantages such as difficulty in use because of poor solubility in solvents, and generating strong odor.

### Citation List

### Patent Literature

Patent Literature 1: Japanese Patent No. 5873770
Patent Literature 2: Japanese Patent No. 5671816
Patent Literature 3: Japanese Patent No. 6505699

### Summary of Invention

### Technical Problem

An object of the present invention is to provide an oxygen-absorbing compound that has oxygen-absorbing properties and generates less odor, and an oxygen-absorbing adhesive composition and a coating agent composition that are produced with the oxygen-absorbing compound.

The oxygen-absorbing adhesive composition is used as an adhesive composition for forming an adhesive layer in a laminate for packaging materials, generates less odor, absorbs oxygen in a packaging bag or packaging container to suppress deterioration of the content due to oxygen, and has an excellent balance between adhesion strength, amount of odor generated, and oxygen-absorbing properties.

The oxygen-absorbing coating agent composition is used as a coating agent composition for forming a coating layer on the surface of a laminate for packaging materials, and is an oxygen-absorbing coating agent composition that generates less odor, absorbs oxygen that permeates the laminate or oxygen inside a packaging bag or packaging container to suppress deterioration of the coating layer, other constituent layers in the laminate, and deterioration of the content due to oxygen, and has an excellent balance between blocking resistance, amount of odor generated, and oxygen-absorbing properties.

### Solution to Problem

Therefore, in order to solve the above problems, the present inventors have found that a specific compound having an unsaturated five-membered ring to which an electron-donating organic group is bonded, an adhesive composition or a coating agent composition containing the oxygen-absorbing compound and an oxidation promoting catalyst, can solve the above problems.

That is, the present invention is characterized in the following points.
1. An oxygen-absorbing compound having one or two or more unsaturated five-membered rings, wherein
   any bond between five carbon atoms constituting the unsaturated five-membered ring is a carbon-carbon double bond,
   a monovalent and/or divalent or higher electron donating organic group 1 is bonded to the unsaturated five-membered ring,
   when there is one unsaturated five-membered ring, the five-membered ring or the organic group 1 has a functional group having active hydrogen or a group in which active hydrogen of the functional group having active hydrogen is replaced with a monovalent organic group 2, and
   when there are two or more unsaturated five-membered rings, the unsaturated five-membered rings are bonded to each other via a divalent or higher organic group 2 that replaces active hydrogen of an active hydrogen group on each of the five-membered rings or the organic group 1.
2. The oxygen-absorbing compound according to 1, wherein a structure of the unsaturated five-membered ring or a structure composed of the unsaturated five-membered ring and the organic group 1 is derived from one or two or more types selected from the group consisting of cyclopentadiene, dicyclopentadiene, norbornene, and derivatives thereof.
3. The oxygen-absorbing compound according to 1 or 2, wherein the organic group 2 includes a structural part derived from an isocyanate-based compound or, an isocyanate-based compound and a hydroxyl group-containing compound.
4. The oxygen-absorbing compound according to 3, wherein the isocyanate-based compound is one or two or more selected from the group consisting of xylylene diisocyanate, hexamethylene diisocyanate, isophorone diisocyanate, toluylene diisocyanate, diphenylmethane diisocyanate, and derivatives thereof.
5. The oxygen-absorbing compound according to 3 or 4, wherein the hydroxyl group-containing compound is one or two or more selected from the group consisting of polyhydric alcohols, polyolefin polyols, polyether polyols, polyester polyols, polycarbonate polyols, poly(meth)acrylic acid ester polyols, phenoxy resins, and urethane chain-extended polyols thereof.
6. The oxygen-absorbing compound according to any of 1 to 5, wherein the organic group 2 has no crosslinkable functional group.
7. The oxygen-absorbing compound according to any of 1 to 5, wherein
   the organic group 2 has one or two or more crosslinkable functional groups, and
   the crosslinkable functional group is a hydroxyl group and/or an isocyanate group.
8. The oxygen-absorbing compound according to any of 1 to 5, wherein the oxygen-absorbing compound contains one or two or more selected from the group consisting of compounds represented by the following formulas (1) to (4) ; 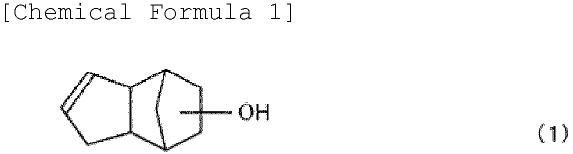 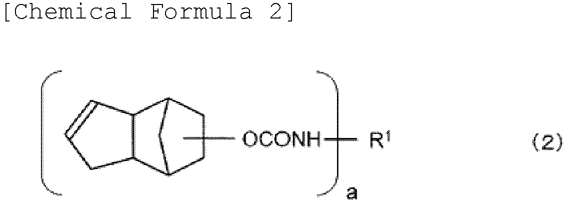 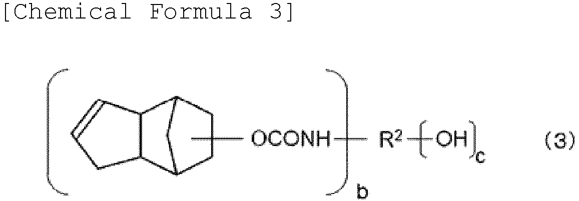 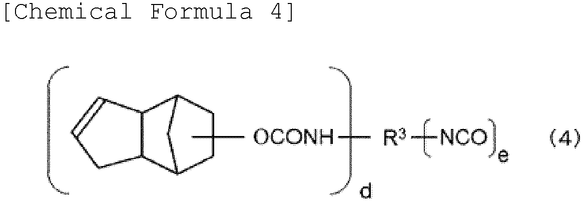 wherein a to e each represent a number of 1 or more, and each of R¹, R², and R³ represents an organic group having 1 or more carbon atoms, includes at least an alkylene and/or phenylene structure, and optionally further includes a structure derived from one or two or more selected from the group consisting of polyhydric alcohols, polyolefin polyols, polyether polyols, polyester polyols, polycarbonate polyols, poly(meth)acrylic acid ester polyols, phenoxy resins, and urethane chain-extended polyols thereof.
9. The oxygen-absorbing compound according to any of 1 to 5, wherein the oxygen-absorbing compound contains a compound represented by the following formula (5): 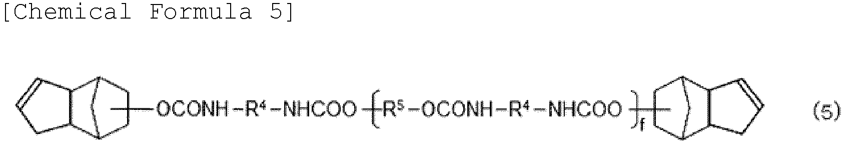 wherein f represents a number of 0 or more, and each of R⁴ and R⁵ represents an organic group having 1 or more carbon atoms, represents an organic group including at least an alkylene and/or phenylene structure, and optionally further includes a structure derived from one or two or more selected from the group consisting of polyhydric alcohols, polyolefin polyols, polyether polyols, polyester polyols, polycarbonate polyols, poly(meth)acrylic acid ester polyols, phenoxy resins, and urethane chain-extended polyols thereof.
10. An oxygen-absorbing composition comprising the oxygen-absorbing compound according to any one of 1 to 9 and an oxidation promoting catalyst.
11. An oxygen-absorbing adhesive composition comprising at least the oxygen-absorbing compound according to any one of 1 to 9 and an oxidation promoting catalyst.
12. The oxygen-absorbing adhesive composition according to 11, wherein the oxidation promoting catalyst is a peroxide or a compound including a cation composed of a transition metal.
13. The oxygen-absorbing adhesive composition according to 12, wherein the compound including a cation composed of a transition metal is a metal soap composed of: a transition metal compound capable of releasing a cation or complex, composed of a transition metal; and an anion or ligand composed of a fatty acid.
14. The oxygen-absorbing adhesive composition according to any of 11 to 13, wherein
   the oxygen-absorbing adhesive composition further contains a modifier, and
   the modifier contains an isocyanate-based compound and/or a hydroxyl group-containing compound.
15. The oxygen-absorbing adhesive composition according to 14, wherein the isocyanate-based compound is one or two or more selected from the group consisting of xylylene diisocyanate, hexamethylene diisocyanate, isophorone diisocyanate, toluylene diisocyanate, diphenylmethane diisocyanate, and derivatives thereof.
16. The oxygen-absorbing adhesive composition according to 14 or 15, wherein the hydroxyl group-containing compound includes one or two or more selected from the group consisting of polyester polyols, poly(meth)acrylic acid ester polyols, polyalkylene ether diols, and urethane chain-extended polyols thereof.
17. An oxygen-absorbing coating agent composition comprising at least the oxygen-absorbing compound according to any one of 1 to 9 and an oxidation promoting catalyst.
18. The oxygen-absorbing coating agent composition according to 17, wherein the oxidation promoting catalyst is a peroxide or a compound including a cation composed of a transition metal.
19. The oxygen-absorbing coating agent composition according to 18, wherein the compound including a cation composed of a transition metal is a metal soap composed of: a transition metal compound capable of releasing a cation or complex, composed of a transition metal; and an anion or ligand composed of a fatty acid.
20. The oxygen-absorbing coating agent composition according to any one of 17 to 19, further comprising a modifier, wherein
   the modifier contains an isocyanate-based compound and/or a hydroxyl group-containing compound.
21. The oxygen-absorbing coating agent composition according to 20, wherein the isocyanate-based compound is one or two or more selected from the group consisting of xylylene diisocyanate, hexamethylene diisocyanate, isophorone diisocyanate, toluylene diisocyanate, diphenylmethane diisocyanate, and derivatives thereof.
22. The oxygen-absorbing coating agent composition according to 20 or 21, wherein the hydroxyl group-containing compound includes one or two or more selected from the group consisting of polyester polyols, poly(meth)acrylic acid ester polyols, polyalkylene ether diols, and urethane chain-extended polyols thereof, vinyl chloride-vinyl acetate copolymers, cellulose acetate-based resins, styrene-maleic acid ester-based copolymers, polyamide-based resins, and polyimide-based resins.

### Advantageous Effects of Invention

The present invention can provide an oxygen-absorbing compound that has oxygen-absorbing properties and generates less odor.

In addition, the present invention can provide an oxygen-absorbing adhesive composition for forming an adhesive layer in a laminate for packaging materials, the composition generating less odor, absorbing oxygen in a packaging bag or packaging container to suppress deterioration of the content due to oxygen, and having an excellent balance between adhesion strength, amount of odor generated, and oxygen-absorbing properties.

The oxygen-absorbing adhesive composition of the present invention is particularly suitable for flexible packaging applications, and achieves shortening the packaging step, reducing costs, and reducing the weight of the package, and eliminating the need to include an oxygen absorber, which has been conventionally included, and thus it is possible to eliminate the accidental ingestion of the oxygen absorber and reduce waste generated by the oxygen absorber.

Further, the present invention can provide an oxygen-absorbing coating agent composition for forming a coating layer on the surface of a film or laminate for packaging materials, the composition generating less odor, absorbing oxygen that permeates the laminate, or oxygen in a packaging bag or packaging container to suppress deterioration of the coating layer and other constituent layers in the laminate and deterioration of the content due to oxygen, and having an excellent balance between blocking resistance, the amount of odor generated, and oxygen-absorbing properties.

The oxygen-absorbing coating agent composition of the present invention is particularly suitable for flexible packaging applications, and achieves shortening the packaging step, reducing costs, and reducing the weight of the package, and eliminating the need to include an oxygen absorber, which has been conventionally included, and thus it is possible to eliminate the accidental ingestion of the oxygen absorber and reduce waste generated by the oxygen absorber.

In addition, a laminate having a coating layer made of the oxygen-absorbing coating agent composition of the present invention on the surface thereof is unlikely to cause blocking even if wound into a roll and stored for a long period of time.

### Brief Description of Drawings

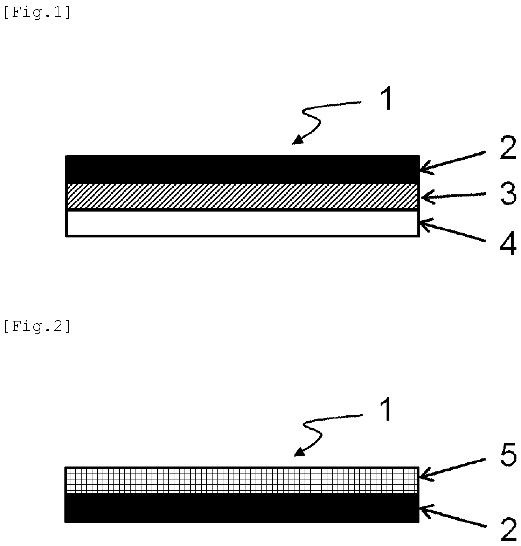

Fig. 1 is a cross-sectional view showing an example of the layer configuration of a laminate with the oxygen-absorbing adhesive composition of the present invention.
Fig. 2 is a cross-sectional view showing an example of the layer configuration of a laminate with the oxygen-absorbing coating agent composition of the present invention.

### Description of Embodiments

The above present invention will be explained in more detail below. The explanation of the constituent elements described below is an example of the embodiment of the present invention, and the present invention is not limited to these contents unless it goes beyond the gist thereof.

### <<Oxygen-absorbing compound>>

The oxygen-absorbing compound of the present invention has oxygen-absorbing properties, generates less odor, and can be used singly or mixed with a resin or resin composition.

The oxygen-absorbing compound of the present invention is an unsaturated five-membered ring-containing compound having one or two or more unsaturated five-membered rings, in which any bond between the five carbon atoms constituting the unsaturated five-membered ring is a carbon-carbon double bond, and a monovalent and/or divalent or higher electron-donating organic group 1 is bonded to the unsaturated five-membered ring.

When there is one unsaturated five-membered ring, the five-membered ring or the organic group 1 has a functional group having active hydrogen, or a group in which the active hydrogen of the functional group having active hydrogen is replaced with a monovalent organic group 2, and when there are two or more unsaturated five-membered rings, the unsaturated five-membered rings are bonded to each other via a divalent or higher organic group 2 that replaces the active hydrogen of the active hydrogen group on each of the organic groups 1.

When there are two or more unsaturated five-membered rings in one molecule, the unsaturated five-membered rings are bonded to each other via a structure in which an active hydrogen of a functional group having an active hydrogen on each of the five-membered rings or organic group 1 is substituted with a divalent or higher organic group 2.

The unsaturated five-membered ring, organic group 1, and organic group 2 present in one molecule may each be of one or two or more types, and the number may be one or two or more. In addition, the number of the organic group 1 bonded to one unsaturated five-membered ring may be one or two or more. Further, the oxygen-absorbing compound may be a mixture of molecules having two or more structures in which the types and numbers of the unsaturated five-membered rings, organic groups 1, and organic groups 2 present in one molecule are different, as described above.

The organic group 1 donates electrons to the unsaturated five-membered ring, whereby the electron density of the carbon-carbon double bond portion of the unsaturated five-membered ring increases, increasing reactivity with oxygen, and improving oxygen absorption.

It is preferable that no electron-withdrawing group is bonded to the unsaturated five-membered ring. Bonding an electron-withdrawing group, the electron density of the carbon-carbon double bond portion of the unsaturated five-membered ring is lowered, reducing the reactivity with oxygen and decreasing the oxygen absorption.

Specific examples of the molecular structure of the oxygen-absorbing compound include one unsaturated five-membered ring bonded to a monovalent or divalent organic group 1, one unsaturated five-membered ring bonded to a monovalent or divalent organic group 1 and a monovalent organic group 2 in this order, two unsaturated five-membered rings bonded via a divalent organic group 1 and a divalent organic group 2, and three unsaturated five-membered rings bonded via a divalent organic group 1 and a trivalent organic group 2.

In addition, the oxygen-absorbing compound may or may not have a crosslinkable functional group.

The crosslinkable functional group may be a functional group that has been possessed by the compound from which the organic group 2 was derived, or may be a functional group that has been added by chemical modification.

The oxygen-absorbing compound has a crosslinkable functional group, whereby the oxygen-absorbing compound is mixed with a resin or a resin composition, the oxygen-absorbing compound becomes more compatible with the resin or the resin composition, or becomes part of the crosslinked structure of the resin or the resin composition, making it less likely to bleed out from the resin or the resin composition or the cured product of the resin composition, and as a result, the content of the oxygen-absorbing compound in the resin or the resin composition can be increased.

Specific examples of the crosslinkable functional groups include aliphatic hydroxyl groups, aromatic hydroxyl groups, isocyanate groups, amino groups, epoxy groups, and (meth) acrylic groups. Among them, the isocyanate groups and aliphatic hydroxyl groups are preferable.

When the oxygen-absorbing compound has a crosslinkable functional group, the number of crosslinkable functional groups contained in one molecule is preferably one or two or more. In addition, the crosslinkable functional groups contained in one molecule may be one type or two or more types.

The functional group equivalent of the crosslinkable functional group is not particularly limited, and is preferably 500 to 20,000, more preferably 1,000 to 15,000, and still more preferably 1,500 to 10,000.

The number average molecular weight of the oxygen-absorbing compound is preferably 100 to 10,000, more preferably 200 to 5,000, and still more preferably 300 to 2,500. If the number average molecular weight is smaller than the above range, precipitation is likely to occur in mixing with a resin or a resin composition. If the number average molecular weight is larger than the above range, mixing with the resin or resin composition increases the viscosity of the mixture, and thus a large amount of diluting solvent tends to be required to be contained, making it difficult to obtain a thick film or layer, and coating suitability tends to deteriorate.

In addition, the oxygen absorption effect of the oxygen-absorbing compound of the present invention can be promoted by heating or adding a catalyst.

### (Unsaturated five-membered ring)

The unsaturated five-membered ring possessed by the oxygen-absorbing compound has a carbon-carbon double bond within the unsaturated five-membered ring. Herein, the carbon-carbon double bond is any bond between five carbon atoms constituting an unsaturated five-membered ring, and there may be one or two in one unsaturated five-membered ring.

The carbon-carbon double bond portion reacts with oxygen molecules in the air and takes in oxygen molecules, causing the oxygen-absorbing compound to exhibit oxygen-absorbing properties.

Examples of the compound from which the unsaturated five-membered ring or the unsaturated five-membered ring to which the electron-donating organic group 1 is bonded is derived include cyclopentadiene, dicyclopentadiene, norbornene, and the derivatives thereof. The oxygen-absorbing compound can have an unsaturated five-membered ring derived from one or two or more selected from the group consisting of these.

The concentration of the unsaturated five-membered ring in the oxygen-absorbing compound is not particularly limited, and is preferably 1% by mass or more and 70% by mass or less, more preferably 5% by mass or more and 60% by mass or less. If the concentration is lower than the above range, the oxygen absorption tends to be insufficient, and it is difficult to obtain an oxygen-absorbing compound having the concentration higher than the above range, and the balance with various physical properties tends to be poor.

### (Electron-donating organic group 1)

Specific examples of the organic group 1 include an alkyl group, an alkylene group, and a cyclic alkylene group. Among them, a cyclic alkylene group is preferable, and one that constitutes an aliphatic bicyclic alkylene group together with the unsaturated five-membered ring is more preferable.

Specific examples of the cyclic alkylene group include a cyclopropylene group, a cyclobutylene group, a cyclopentylene group, a cyclohexylene group, a cycloheptylene group, and a cyclooctylene group. Among them, a cyclopentylene group is more preferable.

### (Electron-withdrawing organic group)

Specific examples of the electron-withdrawing group include phenyl group, phenylene group, carbonyl group, and halogen.

For indene and coumarone, in which only these electron-withdrawing groups are bonded to an unsaturated five-membered ring, the electron density of the carbon-carbon double bond portion of the unsaturated five-membered ring becomes low, reactivity with oxygen decreases, and oxygen absorption is low.

### (Functional group having active hydrogen)

Functional groups having active hydrogen are chemically reactive.

Specific examples of the functional group having active hydrogen include a primary amino group, a secondary amino group, an aliphatic hydroxyl group, an aromatic hydroxyl group, an imino group, a carboxyl group, a urethane group, and an urea group, and among them, the primary amino group, the secondary amino group, the aliphatic hydroxyl group, and the aromatic hydroxyl group are preferable, and the aliphatic hydroxyl group is more preferable.

### (Organic group 2)

The organic group 2 is a monovalent and/or divalent or higher valent group that replaces the active hydrogen of a functional group having active hydrogen on the five-membered ring or organic group 1 and is bonded to the five-membered ring or organic group 1.

When there are two or more unsaturated five-membered rings in one molecule, the unsaturated five-membered rings are bonded to each other via a structure in which the active hydrogen of a functional group having active hydrogen is replaced with the organic group 2 having a valence of two or more.

As a specific example, for example, a functional group having an active hydrogen on the organic group 1 reacts with an isocyanate group of an isocyanate-based compound having a structural part from which the organic group 2 is derived to replace active hydrogen with the organic group 2, allowing bonding by a urethane group.

Bonding the organic group 2 allows the oxygen-absorbing compound to have two or more of the unsaturated five-membered rings in one molecule, further allowing to improve compatibility, dispersibility, and reactivity in mixing with a resin and a resin composition to prepare a mixture. Further, the mixture and the cured product of the mixture can be adjusted to be soft.

The organic group 2 may be an aliphatic group, an aromatic group, or may have both an aliphatic group and an aromatic group.

The organic groups 2 present in one molecule of the oxygen-absorbing compound may be one or two or more types.

The organic group 2 is preferably a group containing a structural part derived from an isocyanate-based compound and/or a hydroxyl group-containing compound. Herein, the structural part derived from an isocyanate-based compound and/or a hydroxyl group-containing compound may include a structural part derived from a reaction product of an isocyanate-based compound and a hydroxyl group-containing compound.

### (Isocyanate-based compound)

Examples of the isocyanate-based compound from which the above organic group 2 is derived include tetramethylene diisocyanate, hexamethylene diisocyanate, lysine diisocyanate, isophorone diisocyanate, norbornane diisocyanate, xylylene diisocyanate, toluylene diisocyanate, diphenylmethane diisocyanate, phenylene diisocyanate, diphenyl ether diisocyanate, hydrogenated diphenylmethane diisocyanate, polymethylene polyphenyl polyisocyanate, and trimethylolpropane adducts thereof, biuret forms thereof, allophanate forms thereof, isocyanurate forms (trimers) thereof, and various derivatives thereof. Among them, biuret forms of toluylene diisocyanate, isophorone diisocyanate, and hexamethylene diisocyanate are preferable.

In the present invention, one or two or more selected from the group consisting of these isocyanate-based compounds can be used as the origin of the structural part of the above monovalent and/or divalent or more hydrocarbon group. When two or more types are used, two types may be used in the same molecule of the oxygen-absorbing compound, or molecules of oxygen-absorbing compounds having one different type may be mixed.

The number average molecular weight of the isocyanate-based compound is preferably 100 to 10,000, more preferably 160 to 5,000. If the number average molecular weight is smaller than the above range, precipitation is likely to occur in mixing with a resin or a resin composition. If the number average molecular weight is larger than the above range, mixing with a resin or a resin composition increases the viscosity of the mixture, and thus a large amount of a diluting solvent tends to be required to be contained, making difficult to obtain a thick film or layer, and coating suitability tends to deteriorate.

### (Hydroxyl group-containing compound)

The hydroxyl group-containing compound is a compound from which the organic group 2 described above is derived, and has two or more hydroxyl groups.

Examples of hydroxyl group-containing compounds include polyhydric alcohols, polyolefin polyols, polyether polyols, polyester polyols, polycarbonate polyols, poly(meth)acrylic acid ester polyols, phenoxy resins, and urethane chain-extended polyols thereof. Among them, the polyether polyols and polyolefin polyols are preferable.

In order to prevent odor generation, it is preferable for the hydroxyl group-containing compound to have no double bonds in the aliphatic chain of the main skeleton or to have two hydroxyl groups. Compounds having terminal hydroxyl groups are preferable because of being more readily available, but hydroxyl groups do not need to be at the terminal.

In the present invention, one or two or more types selected from the group consisting of these can be used as the hydroxyl group-containing compound from which the above organic group 2 is derived. When two or more types are used, two types may be used in the same molecule of the oxygen-absorbing compound, or molecules of the oxygen-absorbing compound having different one type may be mixed.

The number average molecular weight of the hydroxyl group-containing compound is preferably 500 to 10,000, more preferably 750 to 5,000, and still more preferably 1,000 to 3,000. If the number average molecular weight is smaller than the above range, precipitation is likely to occur in mixing with a resin or resin composition. If the number average molecular weight is larger than the above range, mixing with a resin or resin composition increases the viscosity of the mixture, and thus a large amount of diluting solvent tends to be required to be contained, making it difficult to obtain a thick film or layer, and coating suitability tends to deteriorate.

### - Polyhydric alcohols

Polyhydric alcohols are monomers that have two or more hydroxyl groups.

Specific examples of the polyhydric alcohols include diols such as ethylene glycol, propylene glycol, 1,3-propanediol, 1,4-butanediol, 1,3-butanediol, 1,6-hexanediol, cyclohexanedimethanol, 1,8-octanediol, 1,9-nonanediol, neopentyl glycol, 3-methyl-1,5-pentanediol, 2-methyl-1,3-propanediol, 2,2,4-trimethyl-1,3-pentanediol, 2,4-diethyl-1,5-pentanediol, 1,12-octadecanediol, 2,2'-oxydiethanol; glycerin, mannitol, and sorbitol.

Among them, ethylene glycol is preferable from the viewpoint of oxygen absorption.

### - Polyolefin polyol

Polyolefin polyol is a polyolefin-based resin that has two or more hydroxyl groups.

Specific examples of the polyolefin polyol include those not only having a main skeleton of a polyolefin such as polyethylene, polypropylene, polybutylene, polybutadiene, hydrogenated polybutadiene, polyisoprene, hydrogenated polyisoprene, ethylene-vinyl acetate copolymer, ethylene-ethyl (meth)acrylate copolymer, ethylene-(meth)acrylic acid copolymer, and ethylenepropylene copolymer, but also having a hydroxyl group.

Among them, those having an ethylene-vinyl acetate copolymer or hydrogenated polyisoprene as the main skeleton are particularly preferable.

### - Polyether polyol

Polyether polyol is a polyether-based resin having two or more hydroxyl groups.

Polyether polyol is obtained, for example, by dehydration condensation of the above polyhydric alcohols or polyolefin polyols, and has not only a polyether structure in the main skeleton but also a hydroxyl group.

Specific examples of the polyether polyols include diethylene glycol, triethylene glycol, dipropylene glycol, polyethylene ether diol, polypropylene ether diol, and polybutylene ether diol, and glycerin-modified polyether polyols. Among them, polypropylene ether diol is particularly preferable.

### - Polyester polyol

Polyester polyol is a polyester-based resin having two or more hydroxyl groups.

The polyester polyol is obtained, for example, by an esterification reaction between various polyvalent carboxylic acids or derivatives thereof and the above polyhydric alcohols, polyolefin polyols, polyether polyols or the like, and has not only a polyester structure in the main skeleton but also a hydroxyl group.

Specific examples of a polyvalent carboxylic acid include adipic acid, phthalic acid, isophthalic acid, terephthalic acid, maleic acid, fumaric acid, succinic acid, oxalic acid, malonic acid, pimelic acid, azelaic acid, sebacic acid, suberic acid, glutaric acid, 1,4-cyclohexanedicarboxylic acid, and trimellitic acid, and examples of derivatives of these polyvalent carboxylic acids include esterification products, acid anhydrides, and acylation products.

Among them, the polyester polyols using two or more types of polyhydric alcohols and two or more types of polyvalent carboxylic acids in combination are preferable in order to reduce crystallinity.

### - Polycarbonate polyol

Polycarbonate polyol is a polycarbonate-based resin having two or more hydroxyl groups.

Polycarbonate has a polyol-derived portion in the main skeleton, and this polyol-derived portion may be derived from the above polyhydric alcohols, polyolefin polyol, polyether polyol, polyester polyol, and the like.

Among them, the polycarbonate polyol using two or more polyhydric alcohols in combination are preferable in order to reduce crystallinity.

### - Poly(meth)acrylic acid ester polyol

Poly(meth)acrylic acid ester polyol is a (meth)acrylic acid ester (co)polymer having two or more hydroxyl groups.

Poly(meth)acrylic acid ester polyol can be obtained, for example, by using a hydroxyl group-containing monomer such as 2-hydroxyethyl methacrylate or a (meth)acrylic acid ester having a hydroxyl group synthesized from one (meth)acrylic acid or a derivative thereof and one diol, and polymerizing the hydroxyl group-containing monomers with each other or copolymerizing with a (meth)acrylic acid ester not having a hydroxyl group.

The diol that can be used in synthesizing the (meth)acrylic acid ester having a hydroxyl group include the above diols, polyolefin polyol, polyether polyols, and the like.

Among them, the poly(meth)acrylic acid ester copolymer with 2-hydroxyethyl methacrylate is preferable.

### - Phenoxy resin

Phenoxy resin is a resin obtained by reacting a polyhydric phenol compound with a polyhydric epoxy compound, and has a structure in which an aliphatic hydroxyl group is generated at the bond formed by the reaction of an aromatic hydroxyl group with an epoxy group.

As the phenoxy resin, those obtained by reacting a bisphenol with diglycidyl etherified bisphenols are readily available and are common.

Examples of the polyhydric phenol compound include bisphenol A and bisphenol F, and examples of the polyhydric epoxy compound include bisphenol A diglycidyl ether and bisphenol F diglycidyl ether.

Among them, the phenoxy resin with bisphenol A is preferable.

The terminal of the phenoxy resin may be an aromatic hydroxyl group or an epoxy group.

### - Urethane chain-extended polyol

Urethane chain-extended polyol is a polyol having two or more hydroxyl groups obtained by extending the above hydroxyl group-containing compound with a urethane chain.

The urethane chain-extended polyol can be obtained, for example, by subjecting the above various hydroxyl group-containing compounds into polymerization reaction with the above isocyanate-based compounds to extend the urethane chain. In addition, if necessary, diamines or amino alcohols may be used in combination for polymerization.

Among them, there are preferable the urethane chain-extended polyols obtained by reacting the above various hydroxyl group-containing compounds having hydroxyl groups at both ends with diisocyanate-based compounds.

### (Specific examples of oxygen-absorbing compounds)

Specific examples of oxygen-absorbing compounds are shown as follows.

3a,4,5,6,7,7a-Hexahydro-4,7-methano-1H-indenol represented by formula (1), 3a,4,5,6,7,7a-hexahydro-4,7-methano-1H-indenamine represented by formula (1-b), and 3a,4,5,6,7,7a-hexahydro-4,7-methano-1H-inden-1-ol represented by formula (1-c) are examples of oxygen-absorbing compounds having one unsaturated five-membered ring and one organic group 1 in which the one organic group 1 has a hydroxyl group or an amino group as a functional group having active hydrogen.

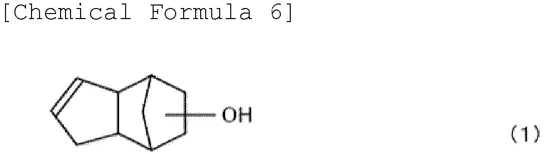

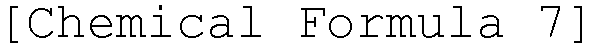

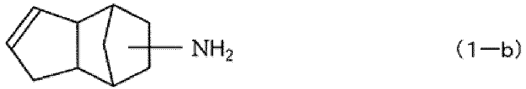

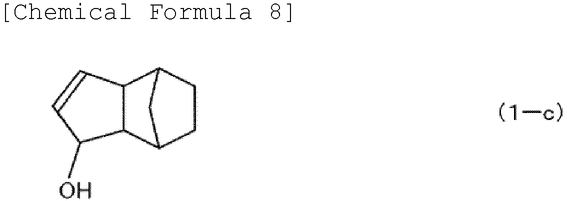

The oxygen-absorbing compound represented by formula (2) is, for example, an oxygen-absorbing compound that can be obtained by reacting a hydroxyl group, which is a functional group having active hydrogen on the organic group 1 of the oxygen-absorbing compound represented by the formula (1), with an isocyanate group of R¹(NCO)ₐ, which is an isocyanate-based compound from which the organic group 2 is derived, to replace the active hydrogen of the hydroxyl group, and bonding a pieces of unsaturated five-membered rings and the organic group 1 via R¹. 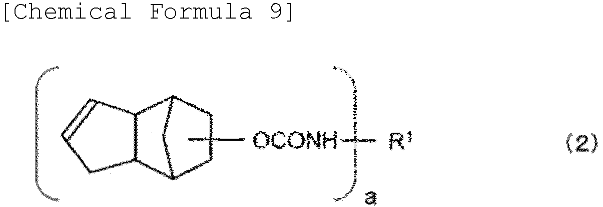 (In the formula, a is a number of 1 or more, R¹ is an organic group having 1 or more carbon atoms, contains at least an alkylene and/or phenylene structure, and can further contain a structure derived from one or two or more selected from the group consisting of polyhydric alcohols, polyolefin polyol, polyether polyol, polyester polyol, polycarbonate polyol, poly(meth)acrylic acid ester polyol, phenoxy resin, and urethane chain-extended polyols thereof.)

The oxygen-absorbing compound represented by formula (5) is an oxygen-absorbing compound in which a = 2 in the formula (2) and R¹ is, for example, a group derived from an isocyanate-based compound OCN-R⁴-NCO and a hydroxyl group-containing compound HO-R⁵-OH, the group containing a structural part formed by the reaction of the two. 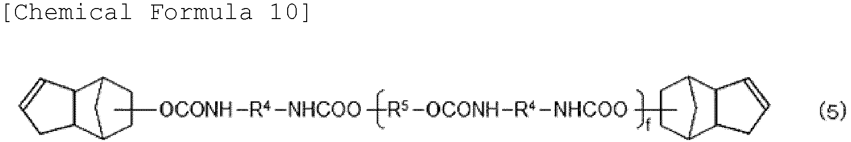 (In the formula, f represents a number of 0 or more, and each of R⁴ and R⁵ represents an organic group having 1 or more carbon atoms, an organic group containing at least an alkylene and/or phenylene structure, and can further contain a structure derived from one or two or more selected from the group consisting of polyhydric alcohols, polyolefin polyols, polyether polyols, polyester polyols, polycarbonate polyols, poly(meth)acrylic acid ester polyols, phenoxy resins, and urethane chain-extended polyols thereof.)

The oxygen-absorbing compound represented by the formula (3) is an oxygen-absorbing compound represented by the formula (2), in which, for example, R¹ is derived from an isocyanate-based compound and a hydroxyl group-containing compound, a structural part formed by the reaction of the two is contained, and hydroxyl groups are contained as residue of excess hydroxyl groups or as a result of chemical modification. 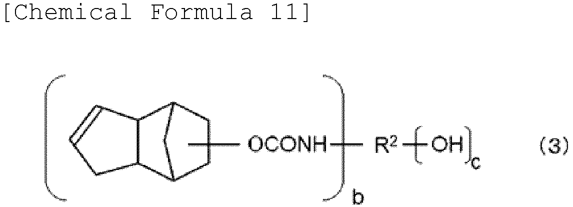 (In the formula, each of b and c represents a number of 1 or more, R² represents an organic group having 1 or more carbon atoms, at least an alkylene and/or phenylene structure is contained, and there can be further contained a structure derived from one or two or more selected from the group consisting of polyhydric alcohols, polyolefin polyols, polyether polyols, polyester polyols, polycarbonate polyols, poly(meth)acrylic acid ester polyols, phenoxy resins, and urethane chain-extended polyols thereof.)

The oxygen-absorbing compound represented by formula (4) is an oxygen-absorbing compound in which in the formula (2), for example, R¹ is derived from an isocyanate-based compound and a hydroxyl group-containing compound, a structural part formed by the reaction of the two is contained, and an isocyanate group is contained due to residue of excess isocyanate groups or chemical modification. 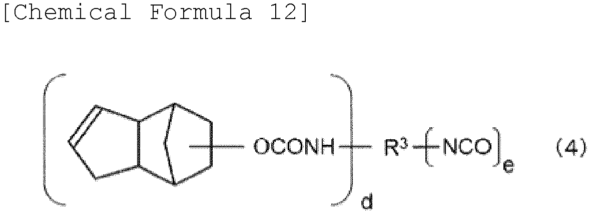 (In the formula, each of d and e represents a number of 1 or more, R³ represents an organic group having 1 or more carbon atoms, at least an alkylene and/or phenylene structure is contained, and there can be further contained a structure derived from one or two or more selected from the group consisting of polyhydric alcohols, polyolefin polyols, polyether polyols, polyester polyols, polycarbonate polyols, poly(meth)acrylic acid ester polyols, phenoxy resins, and urethane chain-extended polyols thereof.)

### <<Oxygen-absorbing composition>>

The oxygen-absorbing composition of the present invention is a composition containing an oxygen-absorbing compound and an oxidation promoting catalyst.

### [Oxidation promoting catalyst]

An oxidation promoting catalyst is a compound that promotes the action of an oxygen-absorbing compound for absorbing oxygen molecules and being oxidized.

Examples of the oxidation promoting catalysts include peroxides and compounds including cations composed of transition metals.

Specific examples of such peroxides include hydrogen peroxide.

The compound including a cation composed of a transition metal is a transition metal-containing compound capable of releasing a cation or complex of a transition metal atom, and is preferably a metal soap composed of a metal salt of a cation and an anion composed of a transition metal.

The transition metal is preferably cobalt, manganese, iron, nickel, copper, or the like, and the anion is preferably an anion composed of stearic acid, naphthenic acid, octylic acid, acetylacetonate, or the like.

As the oxidation promoting catalyst, there can be used a metal soap formed by combining a cation composed of one or two or more transition metals selected from the group consisting of the above transition metals and an anion composed of one or two or more fatty acids selected from the group consisting of the above long-chain fatty acids. Specific examples of the compound include cobalt octylate, acetylacetone cobalt (II), acetylacetone cobalt (III), acetylacetone manganese (III), and acetylacetone iron (III).

### <<Addition of oxygen-absorbing compound to resin or resin composition>>

The resin or resin composition can contain one or two or more types of the oxygen-absorbing compound of the present invention. It is preferable that an oxidation promoting catalyst is further contained. In addition, the resin composition containing the oxygen-absorbing compound of the present invention can contain a diluting solvent and various additives, as in the case of a normal resin composition.

An oxygen-absorbing compound having no crosslinkable functional group may be added to an existing curable resin composition, and an oxygen-absorbing compound having a crosslinkable functional group reactive with a component contained in the existing curable resin composition may be added.

In addition, an oxygen-absorbing compound having a crosslinkable functional group may be used as a main agent or a curing agent in combination with other reactive compounds or resins.

Alternatively, a resin composition may be prepared by combining oxygen-absorbing compounds having two or more types of crosslinkable functional groups so as to crosslink the oxygen-absorbing compounds with each other. Further, an oxygen-absorbing compound having no crosslinkable functional group may be added.

Specifically, for example, an oxygen-absorbing compound without a crosslinkable functional group and/or an oxygen-absorbing compound with a crosslinkable functional group can be added to a urethane-based resin composition containing an isocyanate-based compound and a hydroxyl group-containing compound. The crosslinkable functional group is preferably an isocyanate group and/or a hydroxyl group.

For example, the curable resin composition can be prepared, as the combination of the main agent and the curing agent of the curable resin composition, by combining an isocyanate-based compound and an oxygen-absorbing compound having a hydroxyl group, combining a hydroxyl group-containing compound and an oxygen-absorbing compound having an isocyanate group, or combining an oxygen-absorbing compound having a hydroxyl group and an oxygen-absorbing compound having an isocyanate group.

As the isocyanate-based compound and the hydroxyl group-containing compound used in the curable resin composition, the isocyanate-based compound and the hydroxyl group-containing compound used in the synthesis of the oxygen-absorbing compound as described above can be used.

### <<Oxygen-absorbing adhesive composition>>

The oxygen-absorbing adhesive composition of the present invention is an oxygen-absorbing adhesive composition that contains at least an oxygen-absorbing compound and an oxidation promoting catalyst.

The oxygen-absorbing adhesive composition can further contain a modifier, a diluting solvent, various additives, and the like, if necessary.

The oxygen-absorbing adhesive composition may be prepared by adding an oxygen-absorbing compound to an existing adhesive composition, or the oxygen-absorbing adhesive composition may be prepared with the oxygen-absorbing compound as a resin component.

Herein, the existing oxygen-absorbing adhesive composition may be a one-component oxygen-absorbing adhesive composition or a two-component oxygen-absorbing adhesive composition.

The oxygen-absorbing adhesive composition can contain one or two or more oxygen-absorbing compounds.

The oxygen-absorbing adhesive composition and/or the above existing adhesive composition may be curable or non-curable. If curable, any of heat-curable, photocurable, or electron beam-curable may be possible.

The oxygen-absorbing compound may or may not react with components contained in existing adhesive compositions. In addition, the oxygen-absorbing compounds may react with each other.

Depending on the presence or absence of the above reaction and the type of reaction, one or two or more of oxygen-absorbing compounds can be selected and used from among those having no functional group, those having a (co)polymerizable functional group, and those having a functional group capable of reacting as a main agent or a curing agent.

For example, an oxygen-absorbing adhesive composition can be prepared, as the combination of the main agent and curing agent of the oxygen-absorbing adhesive composition, with a combination of an isocyanate-based compound and an oxygen-absorbing compound having a hydroxyl group, a combination of a hydroxyl group-containing compound and an oxygen-absorbing compound having an isocyanate group, or a combination of an oxygen-absorbing compound having a hydroxyl group and an oxygen-absorbing compound having an isocyanate group.

The oxygen-absorbing adhesive composition is preferably a urethane-based oxygen-absorbing adhesive composition.

Specifically, for example, an oxygen-absorbing compound without a functional group and/or an oxygen-absorbing compound with a functional group can be added to a two-component urethane-based adhesive composition containing an isocyanate-based compound and a hydroxyl group-containing compound. In this case, the functional group is preferably an isocyanate group and/or a hydroxyl group.

There are no particular limitations on the solid content of the oxygen-absorbing adhesive composition, but 20% by mass or more and 100% by mass or less are preferable.

The content of the oxygen-absorbing compound in the solids, excluding the oxidation promoting catalyst, in the oxygen-absorbing adhesive composition is preferably 40% by mass or more and 100% by mass or less. If less than the above range, there is a risk of insufficient oxygen absorption. The case of 100% by mass means that the oxygen-absorbing compound can be used as a resin component of the adhesive composition, and is a case where there is sufficient adhesion singly, there is a curable functional group singly, or an oxygen-absorbing compound having a functional group as a main agent and an oxygen-absorbing compound having a functional group as a curing agent are mixed and used.

The content of the oxidation promoting catalyst in the oxygen-absorbing adhesive composition is preferably 10 ppm or more and 6000 ppm or less with respect to the total solid content.

If the content is less than the above range, oxygen absorption may become insufficient, and if the content is more than the above range, the oxygen absorption tends to become unstable, and the oxygen absorption is lost before the package is produced, which causes a risk of impairing the effect of suppressing deterioration due to oxygen after the package content package is produced.

### [Modifier]

A modifier is a compound having a functional group that reacts with the oxygen-absorbing compound when the oxygen-absorbing compound has a functional group, and various reactive monomers and resins can be used.

Containing the modifier in the oxygen-absorbing adhesive composition can bond the oxygen-absorbing compound to other components in the oxygen-absorbing adhesive composition, adjust the content of the oxygen-absorbing compound in the oxygen-absorbing adhesive composition, and adjust the hardness of the cured product of the oxygen-absorbing adhesive composition.

For example, when the oxygen-absorbing compound has a hydroxyl group or an isocyanate group, a modifier composed of an isocyanate-based compound and/or a hydroxyl group-containing compound can be used.

When the oxygen-absorbing adhesive composition is a urethane-based composition, the equivalent ratio NCO/OH of the oxygen-absorbing adhesive composition is preferably 0.5 or more and 8 or less. At smaller than the above range, the oxygen-absorbing adhesive composition may not cure sufficiently, and sufficient laminate strength (adhesion strength) may not be obtained, and at larger than the above range, the pot life of the oxygen-absorbing adhesive composition may become too short.

### (Isocyanate-based compound for modifier)

As the isocyanate-based compound for the modifier, the isocyanate-based compound used in the synthesis of the oxygen-absorbing compound can be used, and any of aromatic isocyanates, aliphatic isocyanates, and urethane chain-extended isocyanates thereof can be used. In addition, in order for the oxygen-absorbing adhesive composition to be cured, it is preferable to use one having two or more isocyanate groups in one molecule. However, an isocyanate-based compound having one isocyanate group in one molecule can be also used in combination within a range that does not inhibit the sufficient effect of the oxygen-absorbing adhesive composition.

As the isocyanate-based compound having two or more isocyanate groups in one molecule, a biuret form of hexamethylene diisocyanate is particularly preferable.

Specific examples of the isocyanate-based compounds include tetramethylene diisocyanate, hexamethylene diisocyanate, lysine diisocyanate, isophorone diisocyanate, norbornane diisocyanate, xylylene diisocyanate, toluylene diisocyanate, diphenylmethane diisocyanate, hydrogenated diphenylmethane diisocyanate, phenyl diisocyanate, diphenyl ether diisocyanate, polymethylene polyphenyl polyisocyanate, and trimethylolpropane adducts thereof, biuret forms thereof, allophanate forms thereof, isocyanurate forms (trimers) thereof, and further, various derivatives thereof. Among them, biuret forms of toluylene diisocyanate, isophorone diisocyanate, and hexamethylene diisocyanate are preferable.

### (Hydroxyl group-containing compound for modifier)

As the hydroxyl group-containing compound for the modifier, the hydroxyl group-containing compound used in the synthesis of the oxygen-absorbing compound can be used, and any of aromatic hydroxyl group-containing compounds, aliphatic hydroxyl group-containing compounds, and urethane chain-extended polyols thereof can be used. In addition, in order for the oxygen-absorbing adhesive composition to be cured, those having two or more hydroxyl groups in one molecule are preferable. However, a hydroxyl group-containing compound having one hydroxyl group in one molecule can also be used in combination within a range that does not impair the sufficient effect of the oxygen-absorbing adhesive composition.

As the hydroxyl group-containing compound having two or more hydroxyl groups in one molecule, either aromatic hydroxyl group-containing compounds or aliphatic hydroxyl group-containing compounds can be used, and they may be an alcohol-based or a phenol-based.

As the isocyanate-based compound having two or more isocyanate groups in one molecule, polyalkylene ether diols and urethane chain-extended polyols of polyalkylene ether diols are particularly preferable.

### [Diluting solvent]

The diluting solvent is not particularly limited as long as it can uniformly dissolve or disperse the oxygen-absorbing compound and the oxidation promoting catalyst, make the oxygen-absorbing adhesive composition uniform, and is compatible with the dry lamination step, and for example, ester-based diluting solvents, ketone-based diluting solvents, hydrocarbon-based diluting solvents, or the like can be used.

Specific examples of the ester-based diluting solvents include ethyl acetate and butyl acetate, specific examples of the ketone-based diluting solvents include methyl ethyl ketone, and specific examples of the hydrocarbon-based diluting solvents include toluene. Among them, ethyl acetate is easy to use and is preferable.

### [Various additives]

The oxygen-absorbing adhesive composition can contain various additives if necessary.

For example, curing accelerators; curing regulators for extending pot life; and antioxidants, adhesive aids, tackifiers, leveling agents, ultraviolet absorbers, antifoaming agents, coloring pigments, and extender pigments for suppressing a decrease in oxygen absorption during storage or use of an oxygen-absorbing adhesive composition and before the content is accommodated in a package can also be added.

### (Curing accelerator)

A curing accelerator can be used without particular limitations as long as it accelerates the curing reaction of the oxygen-absorbing adhesive composition.

Specific examples of the curing accelerators include metal-containing compounds such as dibutyltin dilaurate, dibutyltin diacetate, dioctyltin dilaurate, dibutyltin dimaleate, tetrabutyl titanate, and tetraisopropyl titanate, and tertiary amines such as 1,8-diaza-bicyclo(5,4,0)undecene-7, 1,5-diazabicyclo(4,3,0)nonene-5 and triethanolamine, and one or two or more types selected from the group consisting of these can be used.

### (Curing regulator)

The oxygen-absorbing adhesive composition is used in combination of a curing regulator to allow the pot life to be extended if the pot life is shortened due to the oxidation promoting catalyst contained.

As specific curing regulators, phosphoric acids are preferable, examples thereof include orthophosphoric acid, metaphosphoric acid, polyphosphoric acid, and ester derivatives thereof, and one or two or more types selected from the group consisting of these can be used.

The amount of the reaction regulator added is preferably 200 ppm or more and 400 ppm or less with respect to the resin component of the oxygen-absorbing adhesive composition. At less than the above range, it is difficult to obtain the effect of extending the pot life, and at more than the above range, there is a risk of inhibiting curing of the oxygen-absorbing adhesive composition.

### (Antioxidant)

In storing or using the oxygen-absorbing adhesive composition, and further, in the step before the package produced with the oxygen-absorbing adhesive composition accommodates the content, the oxygen-absorbing adhesive composition can contain an antioxidant in order to suppress deterioration of oxygen absorption and maintain high oxygen absorption after accommodating the content.

Specific examples of the antioxidant include phenol-based one, lactone-based one, thioether-based one, gallic acid-based one, ascorbic acid, erythorbic acid, catechin, dibutylhydroxytoluene, tocopherol, citric acid, butylhydroxyanisole, phosphite ester, hindered amine, and aromatic amine-based one, and one or two or more types selected from the group consisting of these can be used.

In addition, if it is assumed that heat or light is used as a trigger for developing oxygen absorption, it is preferable to use antioxidants with low heat resistance and light resistance such as ascorbic acid and tocopherol, and it is not preferable to use antioxidants with high heat resistance and high light resistance such as phenol-based one.

The amount of the antioxidant added is preferably 10 ppm or more and 10,000 ppm or less with respect to the oxygen-absorbing compound. At less than the above range, the antioxidant effect tends to be insufficient, and at more than the above range, there is a risk of decreasing the oxygen-absorbing properties.

### (Adhesive aid)

A silane coupling agent is preferable as an adhesive aid to assist in adhesion strength.

Examples of the silane coupling agent include γ-glycidoxypropyltrialkoxysilane, γ-methacryloxypropyltrialkoxysilane, γ-glycidoxypropylmethyldialkoxysilane, β-(3,4-epoxycyclohexyl)ethyltrialkoxysilane, γ-aminopropyltrialkoxysilane, γ-aminopropylmethyldialkoxysilane, N-(β-aminoethyl)-γ-aminopropyltrialkoxysilane, N-(β-aminoethyl)-γ-aminopropylmethyldialkoxysilane, N-butyl-3-amino-2-methylpropyltrialkoxysilane, γ-mercaptopropyltrialkoxysilane, and γ-mercaptopropylmethyldialkoxysilane, and the alkoxy group is preferably a methoxy group or an ethoxy group, and one or two or more types selected from the group consisting of these can be used.

### (Tackifier)

Examples of tackifiers include paraffin wax, polyethylene wax, rosin, rosin glycerin ester, terpene, and alkylphenol, and one or two or more types selected from the group consisting of these can be used.

### (Leveling agent)

Examples of the leveling agent include acrylic polymer-based, modified silicone-based, and acetylene diol-based ones, and one or two or more types selected from the group consisting of these can be used.

### (Ultraviolet absorber)

Examples of ultraviolet absorbers include benzotriazole-based one, hydroxyphenyltriazine-based one, and hindered amine-based one, and one or two or more types selected from the group consisting of these can be used.

### (Antifoaming agent)

Examples of antifoaming agents include surfactants, and polyether-modified silicone oil, and one or two or more types selected from the group consisting of these can be used.

### (Coloring pigment)

Examples of coloring pigments include: organic-based pigments such as anthraquinone, diketopyrrolopyrrole, perylene maroon, carbon black, dioxazine, perylene, benzimidazolone, isoindolinone, isoindoline, phthalocyanine-based, and indanthrene; and inorganic-based pigments such as yellow iron oxide, red iron oxide, azomethine copper complex, titanium oxide, and silicon oxide, and one or two or more types selected from the group consisting of these can be used.

### (Extender pigment)

Examples of extender pigments include white or colorless pigments that are used as fillers or as regulators for coloring power, gloss, strength, feel, and the like.

Specific examples thereof include inorganic-based pigments such as barium sulfate, barium carbonate, calcium carbonate, magnesium oxide, magnesium carbonate, magnesium hydroxide, barium titanate, calcium hydroxide, calcium sulfite, calcium sulfate, calcium oxide, calcium silicate, titanium oxide, silica, zeolite, and talc, and one or two or more types selected from the group consisting of these can be used.

### <<Method for preparing oxygen-absorbing adhesive composition>>

The oxygen-absorbing adhesive composition can be produced by mixing all the constituent components such as an oxygen-absorbing compound, an oxidation promoting catalyst, if necessary, a modifier, a diluting solvent, and various additives. Alternatively, the composition can be produced by mixing an oxygen-absorbing compound, an oxidation promoting catalyst, if necessary, a modifier, a diluting solvent, various additives, or the like into an existing adhesive composition.

The method of performing the above mixing and the order of mixing each component are not particularly limited, and the method and mixing order of preparing a general adhesive composition can be applied.

Specific examples of mixing methods include a method of dissolving and mixing in a solvent and a method of melting and kneading. In this case, it is preferable to adjust the heating temperature to increase solubility and dispersibility.

### <<Method of using oxygen-absorbing adhesive compositions

There is no particular limitation on the method of using the oxygen-absorbing adhesive composition, and the method of using as a general adhesive can be applied.

Examples thereof include a non-solvent lamination method with heating to achieve an appropriate viscosity, and a dry lamination method with diluting solvents and other compounded adhesives to adjust the coating viscosity to an appropriate level.

In a case of forming an adhesive layer using an oxygen-absorbing adhesive composition, the coating amount is preferably 2 to 5 g/m², more preferably 3 to 5 g/m². At less than the above range, there is a risk of failing to obtain sufficient oxygen absorption properties, and at more than the above range, the oxygen absorption properties do not change much, leading to cost disadvantages, which is not preferable.

A laminate obtained by using an oxygen-absorbing adhesive composition to form and adhere an adhesive layer is typically preferably subjected to aging at a temperature of 20°C or more and 50°C or less for 2 days or more and 5 days or less.

During aging, it is preferable to performing aging at the lowest possible temperature or under an inert gas atmosphere so as not to reduce the oxygen absorption properties of the laminate.

In a case of storing the produced laminate, it is preferable to perform storing at 10°C or less or under an inert gas atmosphere so as not to reduce the oxygen absorption properties of the laminate.

### <<Object that can be adhered>>

There are no particular limitations on the object that can be adhered to the oxygen-absorbing adhesive composition, and for example, it is possible to adhere to resin molded products, resin films, paper, metals, metal foils, inorganic vapor-deposited film surfaces, and inorganic oxide vapor-deposited film surfaces.

Specific examples of resins for the resin film include polyester-based resins such as polyethylene terephthalate (PET), polyamide-based resins such as various nylons, polyethylene-based resins, polypropylene-based resins, cyclic polyolefin-based resins, polystyrene-based resins, acrylonitrile-styrene copolymers (AS resins), acrylonitrile-butadiene-styrene copolymers (ABS resins), polyolefin-based resins such as polybutene-based resins, polyvinyl chloride-based resins, polycarbonate-based resins, polyimide-based resins, polyamideimide-based resins, diallyl phthalate-based resins, silicone-based resins, polysulfone-based resins, polyphenylene sulfide-based resins, polyethersulfone-based resins, polyurethane-based resins, cellulose-based resins, poly(meth)acrylic-based resins, polyvinylidene chloride-based resins, acetal-based resins, and fluorine-based resins.

Specific examples of paper include strong sizing bleached or unbleached paper base material for paper layers, or paper base materials such as pure white roll paper, kraft paper, paperboard, coated paper, processed paper, and milk base paper.

Specific examples of the metal foil include aluminum foil, copper foil, and stainless steel foil.

Specific examples of the inorganic materials in the inorganic vapor deposited layer include aluminum.

Specific examples of the inorganic oxides in the inorganic oxide vapor deposited layer include silica, alumina, indium tin oxide, zinc oxide, tin oxide, titanium oxide, zirconium oxide, vanadium oxide, barium oxide, chromium oxide, silicon nitride, and silicon carbide. Among them, silica and alumina are preferable.

### <<Oxygen-absorbing coating agent compositions

The oxygen-absorbing coating agent composition of the present invention is an oxygen-absorbing coating agent composition that contains at least an oxygen-absorbing compound and an oxidation promoting catalyst.

The oxygen-absorbing coating agent composition can further contain a modifier, a diluting solvent, various additives, and the like, if necessary.

The oxygen-absorbing coating agent composition of the present invention can also be used as a printing ink binder.

The oxygen-absorbing coating agent composition may be prepared by adding an oxygen-absorbing compound to an existing coating agent composition, or by using an oxygen-absorbing compound as a resin component.

Herein, the existing coating agent composition may be a one-component coating agent composition or a two-component coating agent composition.

The oxygen-absorbing coating agent composition may contain one or two or more types of oxygen-absorbing compounds.

The oxygen-absorbing coating agent composition and/or the above existing coating agent compositions may be curable or non-curable. If curable, any of heat-curable, photocurable, or electron beam-curable may be possible.

The oxygen-absorbing compound may or may not react with components contained in the existing coating agent compositions. In addition, oxygen-absorbing compounds may react with each other.

Depending on the presence or absence of the above reaction and the type of reaction, one or two or more of oxygen-absorbing compounds can be selected and used from among those having no functional group, those having a (co)polymerizable functional group, and those having a functional group capable of reacting as a main agent or a curing agent.

Specifically, for example, an oxygen-absorbing compound without a functional group and/or an oxygen-absorbing compound with a functional group can be added to a two-component urethane-based coating agent composition containing an isocyanate-based compound and a hydroxyl group-containing compound. In this case, the functional group is preferably an isocyanate group and/or a hydroxyl group.

For example, an oxygen-absorbing coating agent composition can be prepared by, as a combination of the main agent and curing agent of the oxygen-absorbing coating agent composition, combining an isocyanate-based compound and an oxygen-absorbing compound having a hydroxyl group, combining a hydroxyl group-containing compound and an oxygen-absorbing compound having an isocyanate group, or combining an oxygen-absorbing compound having a hydroxyl group and an oxygen-absorbing compound having an isocyanate group.

The oxygen-absorbing coating agent composition is preferably a urethane-based oxygen-absorbing coating agent composition.

The solid content in the oxygen-absorbing coating agent composition is not particularly limited, but is preferably 20% by mass or more and 100% by mass or less.

The content of the oxygen-absorbing compound in the solid, excluding the oxidation promoting catalyst, in the oxygen-absorbing coating agent composition is preferably 30% by mass or more and 100% by mass or less. At less than the above range, there is a risk of insufficient oxygen-absorbing properties. The case of 100% by mass means a case where the oxygen-absorbing compound can be used as a resin component of the coating agent composition, and is a case where there is sufficient coating properties singly, there is a curable functional group singly, or an oxygen-absorbing compound having a functional group as a main agent and an oxygen-absorbing compound having a functional group as a curing agent are mixed and used.

The content of the oxidation promoting catalyst in the oxygen-absorbing coating agent composition is preferably 10 ppm or more and 6000 ppm or less with respect to the total solid content.

If the content is less than the above range, the oxygen absorption may be insufficient, and if the content is more than the above range, the oxygen absorption may become unstable, the oxygen absorption is lost before the package is produced, which causes a risk of impairing the effect of suppressing deterioration due to oxygen after the package content package is produced.

### [Modifier]

The modifier is a compound having a functional group that reacts with the oxygen-absorbing compound when the oxygen-absorbing compound has a functional group, and various reactive monomers and resins can be used.

Containing the modifier in the oxygen-absorbing coating agent composition can bond the oxygen-absorbing compound to other components in the oxygen-absorbing coating agent composition, adjust the content of the oxygen-absorbing compound in the oxygen-absorbing coating agent composition, or adjust the hardness of the cured product of the oxygen-absorbing coating agent composition.

For example, if the oxygen-absorbing compound has a hydroxyl group or an isocyanate group, a modifier composed of an isocyanate-based compound and/or a hydroxyl group-containing compound can be used.

If the oxygen-absorbing coating agent composition is a urethane-based composition, the equivalent ratio NCO/OH of the oxygen-absorbing coating agent composition is preferably 0.5 or more and 8 or less. At smaller than the above range, the oxygen-absorbing coating agent composition does not cure sufficiently, causing a risk of blocking or insufficient adhesion strength with the lower layer, and at larger than the above range, there is a risk of too short pot life of the oxygen-absorbing coating agent composition.

### (Isocyanate-based compound for modifier)

As the isocyanate-based compound for a modifier, an isocyanate-based compound used in the synthesis of the oxygen-absorbing compound can be used, and any of aromatic isocyanates, aliphatic isocyanates, and urethane chain-extended isocyanates thereof can be used. In addition, in order for the oxygen-absorbing coating agent composition to cure, those having two or more isocyanate groups in one molecule are preferable. However, it is also possible to use an isocyanate-based compound having one isocyanate group in one molecule in combination, as long as the sufficient effect of the oxygen-absorbing coating agent composition is not impaired.

As an isocyanate-based compound having two or more isocyanate groups in one molecule, a biuret form of hexamethylene diisocyanate is particularly preferable.

Specific examples of the isocyanate-based compounds include tetramethylene diisocyanate, hexamethylene diisocyanate, lysine diisocyanate, isophorone diisocyanate, norbornane diisocyanate, xylylene diisocyanate, toluylene diisocyanate, diphenylmethane diisocyanate, hydrogenated diphenylmethane diisocyanate, phenylene diisocyanate, diphenyl ether diisocyanate, polymethylene polyphenyl polyisocyanate, and trimethylolpropane adducts thereof, biuret forms thereof, allophanate forms thereof, isocyanurate forms (trimers) thereof, and various derivatives thereof. Among them, biuret forms of toluylene diisocyanate, isophorone diisocyanate, and hexamethylene diisocyanate are preferable.

### (Hydroxyl group-containing compound for modifier)

As the hydroxyl group-containing compound for a modifier, a hydroxyl group-containing compound used in the synthesis of the oxygen-absorbing compound can be used, and any of aromatic hydroxyl group-containing compounds, aliphatic hydroxyl group-containing compounds, and urethane chain-extended polyols thereof can be used. In addition, in order for the oxygen-absorbing coating agent composition to cure, those having two or more hydroxyl groups in one molecule are preferable. However, a hydroxyl group-containing compound having one hydroxyl group in one molecule can also be used in combination as long as the sufficient effect of the oxygen-absorbing coating agent composition is not impaired.

As the hydroxyl group-containing compound having two or more hydroxyl groups in one molecule, either aromatic hydroxyl group-containing compounds or aliphatic hydroxyl group-containing compounds can be used, and they may be alcohol-based or phenol-based.

As the isocyanate-based compound having two or more isocyanate groups in one molecule, polyalkylene ether diols and urethane chain-extended polyols of polyalkylene ether diols are particularly preferable.

### [Diluting solvent]

The diluting solvent is not particularly limited as long as it can uniformly dissolve or disperse an oxygen-absorbing compound and an oxidation promoting catalyst, uniformly form the oxygen-absorbing coating agent composition, and is suitable for coating, and for example, an ester-based diluting solvent, a ketone-based diluting solvent, a hydrocarbon-based diluting solvent, or the like can be used.

Specific examples of the ester-based diluting solvent include ethyl acetate and butyl acetate, specific examples of the ketone-based diluting solvent include methyl ethyl ketone, and specific examples of the hydrocarbon-based diluting solvent include toluene. Among them, ethyl acetate is easy to use and is preferable.

### [Various additives]

The oxygen-absorbing coating agent composition can contain various additives if necessary.

For example, curing accelerators, curing regulators for extending the pot life, antioxidants for suppressing the decrease in oxygen-absorbing properties during storage or use of the oxygen-absorbing coating agent composition or before the content is accommodated in a package, adhesion aids, tackifiers, leveling agents, ultraviolet absorbers, antifoaming agents, coloring pigments, and extender pigments can also be added.

### (Curing accelerator)

The curing accelerator can be used without particular limitations, as long as it accelerates the curing reaction of the oxygen-absorbing coating agent composition.

Specific examples of the curing accelerator include a metal-containing compound such as dibutyltin dilaurate, dibutyltin diacetate, dioctyltin dilaurate, dibutyltin dimaleate, tetrabutyl titanate, and tetraisopropyl titanate, 1,8-diaza-bicyclo(5,4,0)undecene-7, 1,5-diazabicyclo(4,3,0)nonene-5, and a tertiary amine such as triethanolamine, and one or two or more types selected from the group consisting of these can be used.

### (Curing regulator)

If the pot life of an oxygen-absorbing coating agent composition is shortened due to the oxidation promoting catalyst contained, the pot life can be extended by using a curing regulator in combination.

Specific and preferable examples of the curing regulators include phosphoric acids such as orthophosphoric acid, metaphosphoric acid, polyphosphoric acid, and ester derivatives thereof, and one or two or more types selected from the group consisting of these can be used.

The amount of the reaction regulator added is preferably 200 ppm or more and 400 ppm or less with respect to the resin component of the oxygen-absorbing coating agent composition. At less than the above range, it is difficult to obtain the effect of extending the pot life, and at more than the above range, there is a risk of inhibiting the curing of the oxygen-absorbing coating agent composition.

### (Antioxidant)

The oxygen-absorbing coating agent composition can contain an antioxidant in order to suppress deterioration of oxygen-absorbing properties during storage or use of the oxygen-absorbing coating agent composition, and in the step before the package produced with the oxygen-absorbing coating agent composition accommodates the content, and to maintain high oxygen-absorbing properties after the content is accommodated.

Specific examples of the antioxidant include: phenol-based, lactone-based, thioether-based, gallic acid-based, ascorbic acid, erythorbic acid, catechin, dibutylhydroxytoluene, tocopherol, citric acid, butylhydroxyanisole, phosphite ester, hindered amine, and aromatic amine-based one, and one or two or more types selected from the group consisting of these can be used.

In addition, if it is assumed that heat or light is used as a trigger for developing oxygen-absorbing properties, it is preferable to use an antioxidant with low heat resistance and light resistance, such as ascorbic acid and tocopherol, and it is not preferable to use an antioxidant with high heat resistance and high light resistance, such as phenol-based one.

The amount of the antioxidant added is preferably 10 ppm or more and 10,000 ppm or less with respect to the oxygen-absorbing compound. At less than the above range, the antioxidant effect tends to be insufficient, and at more than the above range, there is a risk of decreasing oxygen absorption.

### (Adhesion aid)

A silane coupling agent is preferable as an adhesion aid to assist in adhesion strength.

Examples of the silane coupling agent include γ-glycidoxypropyltrialkoxysilane, γ-methacryloxypropyltrialkoxysilane, γ-glycidoxypropylmethyldialkoxysilane, β-(3,4-epoxycyclohexyl)ethyltrialkoxysilane, γ-aminopropyltrialkoxysilane, γ-aminopropylmethyldialkoxysilane, N-(β-aminoethyl)-γ-aminopropyltrialkoxysilane, N-(β-aminoethyl)-γ-aminopropylmethyldialkoxysilane, N-butyl-3-amino-2-methylpropyltrialkoxysilane, γ-mercaptopropyltrialkoxysilane, and γ-mercaptopropylmethyldialkoxysilane, and as the alkoxy group, a methoxy group or an ethoxy group is preferable, and one or two or more types selected from the group consisting of these can be used.

### (Tackifier)

Examples of the tackifier include paraffin wax, polyethylene wax, rosin, rosin glycerin ester, terpene, and alkylphenol, and one or two or more types selected from the group consisting of these can be used.

### (Leveling agent)

Examples of leveling agents include acrylic polymer-based, modified silicone-based, and acetylene diol-based, and one or two or more types selected from the group consisting of these can be used.

### (Ultraviolet absorber)

Examples of the ultraviolet absorber include benzotriazole-based, hydroxyphenyltriazine-based, and hindered amine-based ones, and one or two or more types selected from the group consisting of these can be used.

Specific example of mixing methods include a method of dissolving and mixing in a solvent and a method of melting and kneading. In this case, it is preferable to adjust the heating temperature to increase solubility and dispersibility.

### <<Method of use of oxygen-absorbing coating agent composition>>

The method of use of the oxygen-absorbing coating agent composition is not particularly limited, and the method of use as a general coating agent can be applied.

For example, a coating layer can be formed by applying once or multiple times with conventionally known means such as a roll coating method, a spray coating method, a spin coating method, a dipping method, a brushing method, a bar coating method, and an applicator method.

When forming a coating layer using an oxygen-absorbing coating agent composition, the coating amount is preferably 2 to 10 g/m², more preferably 3 to 7 g/m². At less than the above range, there is a risk of failing to obtain sufficient oxygen absorption properties, and at more than the above range, the oxygen absorption properties do not change much, leading to cost disadvantages, which is not preferable.

The laminate obtained by forming a coating agent layer using the oxygen-absorbing coating agent composition is preferably aged at a temperature of 20°C or more and 50°C or less for 2 days or more and 5 days or less.

When aging, it is preferable to age at as low a temperature as possible or in an inert gas atmosphere so as not to reduce the oxygen-absorbing properties of the laminate.

When storing the produced laminate, it is also preferable to store at 10°C or less or in an inert gas atmosphere so as not to reduce the oxygen-absorbing properties of the laminate.

### <<Objects that can be coated>>

An object that can be coated with the oxygen-absorbing coating agent composition is not particularly limited, and for example, coating can be performed to resin molded products, resin films, paper, metal, metal foil, inorganic vapor-deposited film surfaces, and inorganic oxide vapor-deposited film surfaces.

Specific examples of the resin for the resin film include polyester-based resins such as polyethylene terephthalate (PET), polyamide-based resins such as various nylons, polyethylene-based resin, polypropylene-based resin, cyclic polyolefin-based resin, polystyrene-based resin, acrylonitrile-styrene copolymer (AS resin), acrylonitrile-butadiene-styrene copolymer (ABS resin), polyolefin-based resins such as polybutene-based resins, polyvinyl chloride-based resin, polycarbonate-based resin, polyimide-based resin, polyamideimide-based resin, diallyl phthalate-based resin, silicone-based resin, polysulfone-based resin, polyphenylene sulfide-based resin, polyether sulfone-based resin, polyurethane-based resin, cellulose-based resin, poly(meth)acrylic-based resin, polyvinylidene chloride-based resin, acetal-based resin, and fluorine-based resin.

Specific examples of paper include paper base materials for paper layers, bleached or unbleached with strong size, or paper base materials such as pure white roll paper, kraft paper, paperboard, coated paper, processed paper, and milk base paper.

Specific examples of metal foil include aluminum foil, copper foil, and stainless steel foil.

Specific examples of the inorganic material in the inorganic vapor deposited layer is aluminum.

Specific examples of the inorganic oxides in the inorganic oxide vapor deposited layer include silica, alumina, indium tin oxide, zinc oxide, tin oxide, titanium oxide, zirconium oxide, vanadium oxide, barium oxide, chromium oxide, silicon nitride, and silicon carbide. Among them, silica and alumina are preferable.

### Example

The present invention will be described in more detail by the following Examples and Comparative Examples, but the present invention is not limited to these Examples.

### <Raw materials>

Main raw materials used in the Examples are as follows.
- Polypropylene ether diol 1: SANNIX PP-1000 produced by Sanyo Chemical Industries, Ltd. Number average molecular weight 1000.
- Polypropylene ether diol 2: SANNIX PK-400, produced by Sanyo Chemical Industries, Ltd. Number average molecular weight 400.
- Hydroxyl group-terminated polyisoprene 1: Poly ip, produced by Idemitsu Kosan Co., Ltd. Number average molecular weight 2500.
- Polycarbonate diol 1: Duranol T5651, produced by Asahi Kasei Chemicals Corporation. Number average molecular weight 1000.
- Toluylene diisocyanate 1: Coronate T-65, produced by Tosoh Corporation.
- Oxygen-absorbing compound 1: 3a,4,5,6,7,7a-hexahydro-4,7-methano-1H-inden-6-ol.
- Oxidation promoting catalyst solution 1: Octope AE, produced by Hope Chemical Co., Ltd. A 4% ethyl acetate solution of cobalt octoate, which is an oxidation promoting catalyst.
- Oxidation promoting catalyst solution 2: Acetope Mn(III), produced by Hope Chemical Co., Ltd. A 10% ethyl acetate solution of manganese (III) acetylacetonate, which is an oxidation promoting catalyst.
- PET film 1: Transparent vapor-deposited PET film (VMPET), IB-PET, produced by Dai Nippon Printing Co., Ltd. 12 µm thick.
- LLDPE film 1: non-stretched linear low-density polyethylene film, N-165, produced by Aicello Corporation 40 µm thick.
- Coating material 1: specially modified polyester resin coating material, Pro Touch White 077-0204, produced by Rock Paint Co., Ltd. Solid content of 49.5 % by mass.
- Coating material 2: acrylic silicone resin coating material, CrystalRock UV Guard Clear, 159-0150, produced by Rock Paint Co., Ltd. Solid content of 47.2 % by mass.
- PET film 1: E5100, produced by Toyobo Co., Ltd.

### <<A. Oxygen-absorbing compound>>

### <Preparation of solution of raw material>

### (Synthesis of polyol A1 and preparation of polyol solution A1)

The following raw materials were added to a flask equipped with a nitrogen inlet tube, a stirrer, a fractionating column and a condenser, and were subjected to a dehydration condensation at an internal temperature of 180 to 200°C while being stirred.

| | |
|---|---|
| Ethylene glycol | 53.8 parts by mass |
| Neopentyl glycol | 180.3 parts by mass |
| 1,6-hexanediol | 204.6 parts by mass |
| Isophthalic acid | 287.8 parts by mass |
| Adipic acid | 273.5 parts by mass |

When the acid number of the solid content in the reaction liquid reached 15 mgKOH/g, the dehydration reaction was further allowed to proceed at 200 to 240°C, while nitrogen was blown in.

When the acid number of the solid content in the reaction liquid reached 10 mgKOH/g or smaller, the internal pressure was reduced to 30 Torr, and the reaction was continued.

When the acid number reached 3 mgKOH/g or smaller, the reaction was finished, the resultant reaction liquid was cooled to room temperature, and a polyol A1 was obtained.

The number average molecular weight of the obtained polyol A1 was 2000, which was a polyester polyol.

Subsequently, the polyol A1 was dissolved in ethyl acetate, the solid content was adjusted to 60% by mass, and a polyol solution A1 was obtained.

### (Synthesis of polyol A2 and preparation of polyol solution A2)

The following raw materials were charged into a flask equipped with a nitrogen inlet tube, a stirrer, and a condenser; and the resultant reaction liquid was heated while being stirred, and subjected to a reflux reaction for 6 hours.

| | |
|---|---|
| Polypropylene ether diol 1 | 300.0 parts by mass |
| Polypropylene ether diol 2 | 250.0 parts by mass |
| Toluylene diisocyanate 1 | 104.0 parts by mass |
| Ethyl acetate | 163.5 parts by mass |

After confirming that the absorption of the isocyanate group completely disappeared in the infrared absorption spectrum, the synthesis was finished, cooling was performed to provide a polyol A2.

The number average molecular weight of the obtained polyol A2 was 2000, which was a urethane chain-extended polyol.

Subsequently, the polyol A2 was dissolved in ethyl acetate, the solid content was adjusted to 60% by mass, and a polyol solution A2 was obtained.

### (Preparation of polyisocyanate solution A1)

The following raw materials were added to a flask equipped with a nitrogen inlet tube, a stirrer and a condenser, the mixture was stirred, and a polyisocyanate solution A1 was obtained which had solid content of 60% by mass.

| | |
|---|---|
| Biuret form of hexamethylene diisocyanate | 100.0 parts by mass |
| Ethyl acetate | 66.7 parts by mass |

**[Table 1]**

| | | Unit | Polyol solution A | | Polyisocyanate solution A |
|---|---|---|---|---|---|
| | | | 1 | 2 | 1 |
| Formulation of reaction liquid | Ethylene glycol | Parts by mass | 53.8 | | |
| | Neopentyl glycol | Parts by mass | 180.3 | | |
| | 1,6-Hexanediol | Parts by mass | 204.6 | | |
| | Polypropylene ether diol 1 | Parts by mass | | 300.0 | |
| | Polypropylene ether diol 2 | Parts by mass | | 250.0 | |
| | Isophthalic acid | Parts by mass | 287.8 | | |
| | Adipic acid | Parts by mass | 273.5 | | |
| | Toluylene diisocyanate 1 | Parts by mass | | 104.0 | |
| | Biuret form of hexamethylene diisocyanate | Parts by mass | | | 100.0 |
| | Ethyl acetate | Parts by mass | | 163.5 | 66.7 |
| | Total | Parts by mass | 1000.0 | 817.5 | 166.7 |
| Additional formulation | Ethyl acetate | Parts by mass | 666.7 | 272.5 | 0.0 |
| | Total | Parts by mass | 1666.7 | 1090.0 | 166.7 |
| Characteristics | Solid content in solution | % by mass | 60.0 | 60.0 | 60.0 |
| | Number average molecular weight | - | 2000 | 2000 | 479 |

### <Synthesis and evaluation of oxygen-absorbing compounds>

### [Example A1]

First, the following raw materials were added to a flask equipped with a nitrogen inlet tube, a stirrer, and a condenser, and were reacted for 8 hours at an internal temperature of 80 to 90°C while being stirred.

| | |
|---|---|
| Oxygen-absorbing compound 1 | 100.0 parts by mass |
| Isophorone diisocyanate | 74.0 parts by mass |

After confirming that the absorption of the isocyanate group had completely disappeared in the infrared absorption spectrum, the synthesis was finished and cooling was performed to provide an oxygen-absorbing compound A2.

Then, the oxygen-absorbing compound A2 was dissolved in ethyl acetate to prepare a solution having a solid content of 80% by mass, and various evaluations were performed.

### [Example A2]

First, the following raw materials were added to a flask equipped with a nitrogen inlet tube, a stirrer, and a condenser, and were reacted for 8 hours at an internal temperature of 80 to 90°C while being stirred.

| | |
|---|---|
| Oxygen-absorbing compound 1 | 100.0 parts by mass |
| Polyisocyanate solution A1 | 212.5 parts by mass |

The content of NCO groups in the solid content of the reaction liquid by the amine equivalent method was confirmed to be approximately 0.64% by mass, then the synthesis was finished, cooling was performed, and the solvent was removed to provide an oxygen-absorbing compound A3.

Then, the oxygen-absorbing compound A3 was dissolved in ethyl acetate to prepare a solution having a solid content of 60% by mass, and various evaluations were performed in the same manner as in Example A1.

### [Example A3]

First, the following raw materials were added to a flask equipped with a nitrogen inlet tube, a stirrer and a condenser, and were reacted for 8 hours at an internal temperature of 80 to 90°C while being stirred.

| | |
|---|---|
| Oxygen-absorbing compound 1 | 100.0 parts by mass |
| Polyol solution A1 | 682.3 parts by mass |
| Polyisocyanate solution A1 | 265.6 parts by mass |

After confirming that the absorption of the isocyanate group had completely disappeared in the infrared absorption spectrum, the synthesis was finished, cooling was performed, and the solvent was removed to provide an oxygen-absorbing compound A4.

Then, the oxygen-absorbing compound A4 was dissolved in ethyl acetate to prepare a solution having a solid content of 60% by mass, and various evaluations were performed in the same manner as in Example A1.

### [Example A4]

An oxygen-absorbing compound A5 was obtained in the same manner as in Example A3, except that the polyol solution A2 was used instead of the polyol solution A1.

Then, the oxygen-absorbing compound A5 was dissolved in ethyl acetate to prepare a solution having a solid content of 60% by mass, and various evaluations were performed in the same manner as in Example A1.

### (Comparative Example A1)

The following raw materials were charged into a flask equipped with a nitrogen inlet tube, a stirrer and a condenser, and were reacted for 8 hours at an internal temperature of 80 to 90°C while being stirred.

| | |
|---|---|
| Hydroxyl group-terminated polyisoprene 1 | 100.0 parts by mass |
| Isophorone diisocyanate | 4.7 parts by mass |

After confirming that the absorption of the isocyanate group had completely disappeared in the infrared absorption spectrum, the synthesis was finished, cooling was performed, and an oxygen-absorbing compound A6 was provided which was a urethane polyol.

The number average molecular weight of the obtained oxygen-absorbing compound A6 was 7000.

Then, the oxygen-absorbing compound A6 was dissolved in ethyl acetate to prepare a solution having a solid content of 60% by mass, and various evaluations were performed in the same manner as in Example A1.

The composition of the reaction liquid charged and the evaluation results in each Example and Comparative Example are summarized in Table 2.

**[Table 2]**

| | | Raw material name | | Unit | Example A | | | | Comparative Example A |
|---|---|---|---|---|---|---|---|---|---|
| | | | | | 1 | 2 | 3 | 4 | 1 |
| Oxygen-absorbing compound reaction liquid | Formulation of raw material | Oxygen-absorbing compound 1 | | Parts by mass | 100.0 | 100.0 | 100.0 | 100.0 | |
| | | Polyol solution A1 | | Parts by mass | | | 682.3 | | |
| | | Polyol solution A2 | | Parts by mass | | | | 682.3 | |
| | | Hydroxyl group-terminated polyisoprene 1 | | Parts by mass | | | | | 100.0 |
| | | Polyisocyanate solution A1 | | Parts by mass | | 212.5 | 265.6 | 265.6 | |
| | | Isophorone diisocyanate | | Parts by mass | 74.0 | | | | 4.7 |
| | | Total | | Parts by mass | 174.0 | 312.5 | 1047.9 | 1047.9 | 104.7 |
| | | Tota solid content | | Parts by mass | 174.0 | 227.5 | 668.7 | 668.7 | 104.7 |
| | | Solid content ratio in reaction liquid | | % by mass | 100.0 | 72.8 | 63.8 | 63.8 | 100.0 |
| | Formulation ratio of solid content | Oxygen-absorbing compound 1 | | % by mass | 57.5 | 44.0 | 15.0 | 15.0 | |
| | | Polyol A1 | | % by mass | | | 61.2 | | |
| | | Polyol A2 | | % by mass | | | | 61.2 | |
| | | Hydroxyl group-terminated polyisoprene 1 | | % by mass | | | | | 95.5 |
| | | Biuret form of hexamethylene diisocyanate | | % by mass | | 56.0 | 23.8 | 23.8 | |
| | | Isophorone diisocyanate | | % by mass | 42.5 | | | | 4.5 |
| | | Total | | % by mass | 100.0 | 100.0 | 100.0 | 100.0 | 100.0 |
| Characteristics | | Equivalent ratio of reaction liquid charged NCO/OH | | - | 1.00 | 1.07 | 0.93 | 0.93 | 0.53 |
| | | Oxygen-absorbing compound | Functional group included | - | Nothing | NCO group | OH group | OH group | OH group |
| | | | Concentration of unsaturated 5-membered ring | % by mass | 25.3 | 19.4 | 6.6 | 6.6 | 0.0 |
| Evaluation result | | Oxygen absorbed amount | | cc/m² | 375 | 290 | 100 | 100 | 600 |
| | | Odor sensory evaluation | | - | 1 | 1 | 1 | 1 | 4 |

### [Summary of results]

The oxygen-absorbing compounds of all the examples of the present invention exhibited excellent oxygen-absorbing properties and low odor.

On the other hand, Comparative Example A1, which is an oxygen-absorbing compound not corresponding to the present invention, exhibited oxygen-absorbing properties but a strong odor.

### <Evaluation method>

### [Production of test film]

A solution containing an oxygen-absorbing compound was applied to a 12 µm-thick PET film with a bar coater so as to provide a dry coating amount of 5.0 g/m², then dried and peeled off to provide a test film of 100 mm × 100 mm.

### [Oxygen absorbed amount]

An oxygen sensor chip (non-destructive oxygen sensor chip manufactured by Precision Sensing GmbH) and the test film produced above were enclosed inside a glass bottle with a capacity of 106 cc.

The oxygen concentration was measured immediately after enclosing and after 14 days of storage in a thermostatic chamber at 25°C, and the oxygen absorbed amount of the test film was calculated. The oxygen concentration was measured with the use of a non-destructive oxygen concentration meter (FIBOX4 OXYGEN METER) manufactured by Presence GmbH.

### [Odor sensory evaluation]

After measuring the oxygen absorbed amount, the odor of the air inside the glass bottle was evaluated sensorily according to the following criteria.

### Evaluation criteria:

0: no odor
1: faint odor
2: weak odor
3: medium odor
4: strong odor

### <<B. Oxygen-absorbing adhesive compositions

The oxygen-absorbing compounds A2 to A6 synthesized above were dissolved in ethyl acetate to prepare oxygen-absorbing compound solutions B2 to B6 having a solid content of 80% by mass or 60% by mass.

The compositions of the oxygen-absorbing compound solutions B1 to B6 charged are summarized in Table 3.

**[Table 3]**

| | Raw material name | Unit | Oxygen-absorbing compound solution B | | | | |
|---|---|---|---|---|---|---|---|
| | | | For Examples | | | | For Comparative Example |
| | | | 2 | 3 | 4 | 5 | 6 |
| Formulation of oxygen-absorbing compound solution | Oxygen-absorbing compound A2 | Parts by mass | 300 | | | | |
| | Oxygen-absorbing compound A3 | Parts by mass | | 300 | | | |
| | Oxygen-absorbing compound A4 | Parts by mass | | | 300 | | |
| | Oxygen-absorbing compound A5 | Parts by mass | | | | 300 | |
| | Oxygen-absorbing compound A6 | Parts by mass | | | | | 300 |
| | Ethyl acetate | Parts by mass | 75 | 200 | 200 | 200 | 200 |
| | Total | Parts by mass | 375 | 500 | 500 | 500 | 500 |
| Characteristics | Content ratio of oxygen-absorbing compound in solution | % by mass | 80.0 | 60.0 | 60.0 | 60.0 | 60.0 |

### <Preparation and evaluation of oxygen-absorbing adhesive composition>

### [Example B1]

The following raw materials were mixed and homogenized at room temperature to provide an oxygen-absorbing adhesive composition, which was then subjected to various evaluations. The evaluation results are shown in Table 4.
Oxygen-absorbing compound solution B2 100 parts by mass
Polyol solution A1 100 parts by mass
Polyisocyanate solution A1 10 parts by mass
Oxidation promoting catalyst solution 1 1 part by mass
Ethyl acetate 140 parts by mass

### [Examples B2 to B9, Comparative Examples B1 and B2]

According to the formulations in Tables 5 and 6, oxygen-absorbing adhesive compositions were provided in the same manner as in Example B1, and various evaluations were similarly performed. The evaluation results are shown in Tables 4 and 5.

**[Table 4]**

| | | | Unit | Example B | | | | |
|---|---|---|---|---|---|---|---|---|
| | | | | 1 | 2 | 3 | 4 | 5 |
| Formulation of adhesive composition | Oxygen-absorbing compound | Oxygen-absorbing compound solution B2 | Parts by mass | 100.0 | | | | |
| | | Oxygen-absorbing compound solution B3 | Parts by mass | | 100.0 | | | |
| | | Oxygen-absorbing compound solution B4 | Parts by mass | | | 250.0 | | 125.0 |
| | | Oxygen-absorbing compound solution B5 | Parts by mass | | | | 250.0 | 125.0 |
| | | Oxygen-absorbing compound solution B6 | Parts by mass | | | | | |
| | Hydroxyl-group containing compound | Polyol solution A1 | Parts by mass | 100.0 | 100.0 | | | |
| | | Polyol solution A2 | Parts by mass | | | | | |
| | Isocyanate-based compound | Polyisocyanate solution A1 | Parts by mass | 10.0 | | 25.0 | 25.0 | 25.0 |
| | Oxidation promoting catalyst | Oxidation promoting catalyst solution 1 | Parts by mass | 1.0 | 1.0 | 1.0 | 1.0 | 1.0 |
| | | Oxidation promoting catalyst solution 2 | Parts by mass | | | | | |
| | Solvent | Ethyl acetate | Parts by mass | 140.0 | 140.0 | 220.0 | | 110.0 |
| | Total | | Parts by mass | 351.0 | 341.0 | 496.0 | 276.0 | 386.0 |
| Composition of raw material solid content of adhesive composition | Oxygen-absorbing compound | Oxygen-absorbing compound A2 | % by mass | 54.8 | | | | |
| | | Oxygen-absorbing compound A3 | % by mass | | 50.0 | | | |
| | | Oxygen-absorbing compound A4 | % by mass | | | 90.9 | | 45.5 |
| | | Oxygen-absorbing compound A5 | % by mass | | | | 90.9 | 45.5 |
| | | Oxygen-absorbing compound A6 | % by mass | | | | | |
| | Hydroxyl-group containing compound | Polyol A1 | % by mass | 41.1 | 50.0 | | | |
| | | Polyol A2 | % by mass | | | | | |
| | Isocyanate-based compound | Biuret form of hexamethylene diisocyanate | % by mass | 4.1 | | 9.1 | 9.1 | 9.1 |
| | Oxidation promoting catalyst | Oxidation promoting catalyst 1 | ppm | 274 | 333 | 242 | 242 | 242 |
| | | Oxidation promoting catalyst 2 | ppm | | | | | |
| | Total | | % by mass | 100.0 | 100.0 | 100.0 | 100.0 | 100.0 |
| Evaluation result | Oxygen absorbed amount | | cc/m² | 210 | 185 | 92 | 92 | 92 |
| | Laminate strength | | N/15mm | 4.2 | 4.5 | 4.6 | 4.6 | 4.6 |
| | Odor sensory evaluation | | - | 1 | 1 | 1 | 1 | 1 |

**[Table 5]**

| | | | Unit | Example B | | | | Comparative Example B | |
|---|---|---|---|---|---|---|---|---|---|
| | | | | 6 | 7 | 8 | 9 | 1 | 2 |
| Formulation of adhesive composition | Oxygen-absorbing compound | Oxygen-absorbing compound solution B2 | Parts by mass | | | | | | |
| | | Oxygen-absorbing compound solution B3 | Parts by mass | 100.0 | 100.0 | 100.0 | 100.0 | | |
| | | Oxygen-absorbing compound solution B4 | Parts by mass | 100.0 | | | 100.0 | | |
| | | Oxygen-absorbing compound solution B5 | Parts by mass | | 100.0 | | | | |
| | | Oxygen-absorbing compound solution B6 | Parts by mass | | | | | 100.0 | |
| | Hydroxyl-group containing compound | Polyol solution A1 | Parts by mass | | | | | | 250.0 |
| | | Polyol solution A2 | Parts by mass | | | 100.0 | | | |
| | Isocyanate-based compound | Polyisocyanate solution A1 | Parts by mass | | | | | 25.0 | 25.0 |
| | Oxidation promoting catalyst | Oxidation promoting catalyst solution 1 | Parts by mass | 1.0 | 1.0 | 1.0 | | 1.5 | |
| | | Oxidation promoting catalyst solution 2 | Parts by mass | | | | 2.0 | | |
| | Solvent | Ethyl acetate | Parts by mass | | | 140.0 | | | |
| | Total | | Parts by mass | 201.0 | 201.0 | 341.0 | 202.0 | 126.5 | 275.0 |
| Composition of raw material solid content of adhesive composition | Oxygen-absorbing compound | Oxygen-absorbing compound A2 | % by mass | | | | | | |
| | | Oxygen-absorbing compound A3 | % by mass | 50.0 | 50.0 | 50.0 | 50.0 | | |
| | | Oxygen-absorbing compound A4 | % by mass | 50.0 | | | 50.0 | | |
| | | Oxygen-absorbing compound A5 | % by mass | | 50.0 | | | | |
| | | Oxygen-absorbing compound A6 | % by mass | | | | | 80.0 | |
| | Hydroxyl-group containing compound | Polyol A1 | % by mass | | | | | | 90.9 |
| | | Polyol A2 | % by mass | | | 50.0 | | | |
| | Isocyanate-based compound | Biuret form of hexamethylene diisocyanate | % by mass | | | | | 20.0 | 9.1 |
| | Oxidation promoting catalyst | Oxidation promoting catalyst 1 | ppm | 333 | 333 | 333 | | 799 | |
| | | Oxidation promoting catalyst 2 | ppm | | | | 1664 | | |
| | Total | | % by mass | 100.0 | 100.0 | 100.0 | 100.0 | 100.0 | 100.0 |
| Evaluation result | Oxygen absorbed amount | | cc/m² | 215 | 215 | 185 | 150 | 600 | 0 |
| | Laminate strength | | N/15mm | 4.1 | 4.1 | 4.4 | 4.1 | 4.1 | 4.2 |
| | Odor sensory evaluation | | - | 1 | 1 | 1 | 1 | 4 | 0 |

### [Summary of results]

The oxygen-absorbing adhesive compositions of all the examples of the present invention exhibited a balance of excellent oxygen-absorbing properties, adhesion, and low odor.

On the other hand, the adhesive composition of Comparative Example B1, which was prepared with an oxygen-absorbing compound different from that of the present invention and did not contain an oxygen-absorbing compound, exhibited a strong odor, and the adhesive composition of Comparative Example B2 did not exhibit oxygen-absorbing properties.

### <Evaluation method>

### [Production of laminate film for test pieces]

The adhesive composition was applied to PET film 1 with a bar coater and then dried so as to provide a dry coating amount of 5.0 g/m², LLDPE film 1 was attached thereon and nipped on a hot plate at 60°C to provide a laminate film for test pieces, which was then aged at 25°C for 2 days.

### [Oxygen absorbed amount]

Using the laminate film for test pieces obtained above, there was prepared a packaging bag in which an oxygen sensor chip (non-destructive oxygen sensor chip, manufactured by PreSens Precision Sensing GmbH) was enclosed.

The packaging bag was produced by folding the laminate film in half and heat sealing three sides so as to provide internal dimensions of 130 mm × 70 mm.

Then, 26 cc of air was injected into the packaging bag with a syringe and the injected portion was repaired with adhesive tape, and the oxygen concentration was measured at the time of injection and after 14 days of storage in a thermostatic chamber at 25°C to calculate the oxygen absorbed amount.

### [Laminate strength evaluation]

A strip-shaped test piece having a width of 15 mm was produced from the laminate film for test pieces obtained above, and for the laminate strength between PET film 1 and LLDPE film 1, the laminate strength was measured with a tensile tester at a tensile speed of 50 mm/min.

### [Odor sensory evaluation]

After measuring the oxygen-absorbing properties, the packaging bag was opened and the odor was evaluated sensorily according to the following criteria.

### Evaluation criteria:

0: no odor
1: faint odor
2: weak odor
3: medium odor
4: strong odor

### <<C. Oxygen-absorbing coating agent compositions

### <Preparation of raw material solution>

### (Synthesis of polyol C1 and preparation of polyol solution C1)

The following raw materials were added to a flask equipped with a nitrogen inlet tube, a stirrer, a fractionating column and a condenser, a dehydration condensation reaction was allowed to proceed at an internal temperature of 180 to 200°C while being stirred.

| | |
|---|---|
| Ethylene glycol | 70.0 parts by mass |
| Neopentyl glycol | 140.0 parts by mass |
| 1,6-hexanediol | 190.0 parts by mass |
| Isophthalic acid | 300.0 parts by mass |
| Terephthalic acid | 300.0 parts by mass |

When the acid number of the solid content in the reaction liquid reached 15 mgKOH/g, the dehydration reaction was further allowed to proceed at 200 to 240°C, while nitrogen was blown in.

When the acid number of the solid content in the reaction liquid reached 10 mgKOH/g or smaller, the internal pressure was reduced to 30 Torr, and the reaction was continued.

When the acid number of the solid content in the reaction liquid became 0.1 mgKOH/g or less, the reaction was finished and cooling was performed to room temperature to provide polyol C1.

The number average molecular weight of the resulting polyester polyol, polyol C1, was 8,000.

Then, the polyol C1 was dissolved in ethyl acetate to adjust the solid content to 60% by mass, to provide polyol solution C1.

### (Synthesis of polyol C2 and preparation of polyol solution C2)

The following raw materials were charged into a flask equipped with a nitrogen inlet tube, a stirrer, and a condenser; and the mixture was heated while being stirred, and subjected to a reflux reaction for 6 hours.

| | |
|---|---|
| Polycarbonate diol 1 | 100.0 parts by mass |
| 1,6-hexanediol | 50.1 parts by mass |
| Isophorone diisocyanate | 118.5 parts by mass |
| Ethyl acetate | 138.0 parts by mass |

After confirming that the absorption of the isocyanate groups had completely disappeared with an infrared absorption spectrum, the synthesis was finished and cooling was performed to provide polyol C2.

The number average molecular weight of the resulting urethane chain-extended polyester-polycarbonate polyol, polyol C2, was 8,000.

Then, the polyol C2 was dissolved in ethyl acetate so as to prepare a solids content of 60% by mass, to provide polyol solution C2.

The following raw materials were added to a flask equipped with a nitrogen inlet tube, a stirrer, a fractionating column and a condenser, a dehydration condensation reaction was allowed to proceed at an internal temperature of 180 to 200°C while being stirred.

| | |
|---|---|
| Ethylene glycol | 70.0 parts by mass |
| Neopentyl glycol | 140.0 parts by mass |
| 1,6-hexanediol | 190.0 parts by mass |
| Isophthalic acid | 300.0 parts by mass |
| Terephthalic acid | 300.0 parts by mass |

When the acid number of the solid content in the reaction liquid reached 15 mgKOH/g, the dehydration reaction was further allowed to proceed at 200 to 240°C, while nitrogen was blown in.

When the acid number of the solid content in the reaction liquid reached 10 mgKOH/g or smaller, the internal pressure was reduced to 30 Torr, and the reaction was continued.

When the acid number of the solid content in the reaction liquid reached 3 mgKOH/g or less, the reaction was finished and cooling was performed to room temperature to provide polyol C3.

The number average molecular weight of the resulting polyester polyol, polyol C3, was 2000.

Then, the polyol C3 was dissolved in ethyl acetate to adjust the solids content to 60% by mass, to provide polyol solution C3.

### (Preparation of polyisocyanate solution C1)

The following raw materials were added to a flask equipped with a nitrogen inlet tube, a stirrer, and a condenser, and stirred to provide a polyisocyanate solution C1 having a solid content of 60% by mass.

| | |
|---|---|
| Hexamethylene diisocyanate biuret form | 100.0 parts by mass |
| Ethyl acetate | 66.7 parts by mass |

**[Table 6]**

| | | Unit | Polyol solution C | | | Polyisocyanate solution C |
|---|---|---|---|---|---|---|
| | | | 1 | 2 | 3 | 1 |
| Formulation of reaction liquid | Ethylene glycol | Parts by mass | 70.0 | | 70.0 | |
| | Neopentyl glycol | Parts by mass | 140.0 | | 140.0 | |
| | 1,6-Hexanediol | Parts by mass | 190.0 | 50.1 | 190.0 | |
| | Polycarbonate diol 1 | Parts by mass | | 100.0 | | |
| | Isophthalic acid | Parts by mass | 300.0 | | 300.0 | |
| | Terephthalic acid | Parts by mass | 300.0 | | 300.0 | |
| | Isophorone diisocyanate | Parts by mass | | 118.5 | | |
| | Biuret form of hexamethylene diisocyanate | Parts by mass | | | | 100.0 |
| | Ethyl acetate | Parts by mass | | 138.0 | | 66.7 |
| | Total | Parts by mass | 1000.0 | 406.6 | 1000.0 | 166.7 |
| Additional formulation | Ethyl acetate | Parts by mass | 666.7 | 41.0 | 666.7 | 0.0 |
| | Total | Parts by mass | 1666.7 | 447.6 | 1666.7 | 166.7 |
| Characteristics | Solid content in solution | % by mass | 60.0 | 60.0 | 60.0 | 60.0 |
| | Number average molecular weight | - | 8000 | 8000 | 2000 | 479 |

### <Synthesis of oxygen-absorbing compound and preparation of oxygen-absorbing compound solution>

### [Oxygen-absorbing compound C2]

First, the following raw materials were added to a flask equipped with a nitrogen inlet tube, a stirrer and a condenser, and were reacted for 8 hours at an internal temperature of 80 to 90°C while being stirred.

| | |
|---|---|
| Oxygen-absorbing compound 1 | 100.0 parts by mass |
| Isophorone diisocyanate | 74.0 parts by mass |

After confirming that the absorption of the isocyanate group completely disappeared in the infrared absorption spectrum, the synthesis was finished, cooling was performed to provide an oxygen-absorbing compound 2.

Then, the oxygen-absorbing compound 2 was dissolved in ethyl acetate to prepare oxygen-absorbing compound solution C2 having a solid content of 80% by mass.

### [Oxygen-absorbing compound C3]

First, the following raw materials were added to a flask equipped with a nitrogen inlet tube, a stirrer and a condenser, and were reacted for 8 hours at an internal temperature of 80 to 90°C while being stirred.

| | |
|---|---|
| Oxygen-absorbing compound 1 | 100.0 parts by mass |
| Polyisocyanate solution 1 | 212.5 parts by mass |

The synthesis was finished when it was confirmed that the NCO group content in the solid content of the reaction liquid by the amine equivalent method was approximately 0.64% by mass, cooling was performed, and the solvent was removed to provide an oxygen-absorbing compound C3.

Then, the oxygen-absorbing compound C3 was dissolved in ethyl acetate to prepare an oxygen-absorbing compound solution C3 having a solid content of 60% by mass.

### [Oxygen-absorbing compound C4]

First, the following raw materials were added to a flask equipped with a nitrogen inlet tube, a stirrer and a condenser, and were reacted for 8 hours at an internal temperature of 80 to 90°C while being stirred.

| | |
|---|---|
| Oxygen-absorbing compound 1 | 100.0 parts by mass |
| Polyol solution 1 | 2000.0 parts by mass |
| Isophorone diisocyanate | 111.2 parts by mass |

After confirming that the absorption of the isocyanate group completely disappeared in the infrared absorption spectrum, the synthesis was finished, cooling was performed, the solvent was removed to provide an oxygen-absorbing compound C4.

Then, the oxygen-absorbing compound C4 was dissolved in ethyl acetate, and an oxygen-absorbing compound solution C4 was prepared which had solid content of 60% by mass.

### [Oxygen-absorbing compound C5]

First, the following raw materials were added to a flask equipped with a nitrogen inlet tube, a stirrer and a condenser, and were reacted for 8 hours at an internal temperature of 80 to 90°C while being stirred.

| | |
|---|---|
| Oxygen-absorbing compound 1 | 100.0 parts by mass |
| Polyol solution 3 | 682.3 parts by mass |
| Polyisocyanate solution 1 | 265.6 parts by mass |

After confirming that the absorption of the isocyanate group completely disappeared in the infrared absorption spectrum, the synthesis was finished, cooling was performed, the solvent was removed to provide an oxygen-absorbing compound 5.

Then, the oxygen-absorbing compound 5 was dissolved in ethyl acetate, and an oxygen-absorbing compound solution C5 was prepared which had solid content of 60% by mass.

The charged compositions of the reaction liquids at the time when the oxygen-absorbing compounds C2 to C5 were synthesized are summarized in Table 7.

In addition, the charged compositions of the oxygen-absorbing compound solutions C2 to C5 are summarized in Table 8.

**[Table 7]**

| | | Raw material name | | Unit | Oxygen-absorbing compound C | | | |
|---|---|---|---|---|---|---|---|---|
| | | | | | 2 | 3 | 4 | 5 |
| Oxygen-absorbing compound reaction liquid | Formulation of raw material | Oxygen-absorbing compound 1 | | Parts by mass | 100.0 | 100.0 | 100.0 | 100.0 |
| | | Polyol solution C1 | | Parts by mass | | | 2000.0 | |
| | | Polyol solution C3 | | Parts by mass | | | | 682.3 |
| | | Polyisocyanate solution C1 | | Parts by mass | | 212.5 | | 265.6 |
| | | Isophorone diisocyanate | | Parts by mass | 74.0 | | 111.2 | |
| | | Total | | Parts by mass | 174.0 | 312.5 | 2211.2 | 1047.9 |
| | | Solid content ratio in reaction liquid | | % by mass | 100.0 | 72.8 | 63.8 | 63.8 |
| | Formulation ratio of solid content | Oxygen-absorbing compound 1 | | % by mass | 57.5 | 44.0 | 7.1 | 15.0 |
| | | Polyol C1 | | % by mass | | | 85.0 | |
| | | Polyol C3 | | % by mass | | | | 61.2 |
| | | Biuret form of hexamethylene diisocyanate | | % by mass | | 56.0 | | 23.8 |
| | | Isophorone diisocyanate | | % by mass | 42.5 | | 7.9 | |
| | | Total | | % by mass | 100.0 | 100.0 | 100.0 | 100.0 |
| Characteristics | | Equivalent ratio of reaction liquid charged NCO/OH | | - | 1.00 | 1.20 | 1.04 | 0.93 |
| | | Oxygen-absorbing compound | Functional group included | | Nothing | NCO group | NCO group | OH group |
| | | | Concentration of unsaturated 5-membered ring | % by mass | 25.3 | 19.4 | 3.1 | 6.6 |

**[Table 8]**

| | Raw material name | Unit | compound solution c | | | |
|---|---|---|---|---|---|---|
| | | | 2 | 3 | 4 | 5 |
| Formulation of oxygen-absorbing compound solution | Oxygen-absorbing compound C2 | Parts by mass | 300 | | | |
| | Oxygen-absorbing compound C3 | Parts by mass | | 300 | | |
| | Oxygen-absorbing compound C4 | Parts by mass | | | 300 | |
| | Oxygen-absorbing compound C5 | Parts by mass | | | | 300 |
| | Ethyl acetate | Parts by mass | 75 | 200 | 200 | 200 |
| | Total | Parts by mass | 375 | 500 | 500 | 500 |
| Characteristics | Content ratio of oxygen-absorbing compound in solution | % by mass | 80.0 | 60.0 | 60.0 | 60.0 |

### <Preparation and evaluation of oxygen-absorbing coating agent composition>

### [Example C1]

The following raw materials were mixed at room temperature to provide an oxygen-absorbing coating agent composition, which was then subjected to various evaluations.

| | |
|---|---|
| Oxygen-absorbing compound solution C2 | 100.0 parts by mass |
| Coating material 1 | 192.0 parts by mass |
| Oxidation promoting catalyst solution 1 | 1.1 parts by mass |

### [Examples C2 to C9 and Comparative Example C1]

According to the formulation in the table, an oxygen-absorbing coating agent composition was obtained in the same manner as in Example C1, and various evaluations were carried out in the same manner. However, in Examples 8 and 9, after coating and drying, aging was performed at 40°C for 72 hours. The evaluation results are shown in Tables 9 and 10.

**[Table 9]**

| | | Unit | Example C | | | | |
|---|---|---|---|---|---|---|---|
| | | | 1 | 2 | 3 | 4 | 5 |
| Formulation of coating agent composition | Oxygen-absorbing compound solution C2 | Parts by mass | 100.0 | 100.0 | | | |
| | Oxygen-absorbing compound solution C3 | Parts by mass | | | 100.0 | 100.0 | 100.0 |
| | Oxygen-absorbing compound solution C4 | Parts by mass | | | | | |
| | Oxygen-absorbing compound solution C5 | Parts by mass | | | | | |
| | Paint 1 | Parts by mass | 192.0 | | 192.0 | | |
| | Paint 2 | Parts by mass | | 201.0 | | 201.0 | |
| | Polyol solution C1 | Parts by mass | | | | | 158.0 |
| | Polyol solution C2 | Parts by mass | | | | | |
| | Hydroxyl group-terminated polyisoprene 1 | Parts by mass | | | | | |
| | Polyisocyanate solution C1 | Parts by mass | | | | | |
| | Oxidation promoting catalyst solution 1 | Parts by mass | 1.10 | 1.10 | 1.00 | 1.00 | 1.00 |
| | Oxidation promoting catalyst solution 2 | Parts by mass | | | | | |
| | Total | Parts by mass | 293.1 | 302.1 | 293.0 | 302.0 | 259.0 |
| | Solid content (%) | % by mass | 59.7 | 57.9 | 52.9 | 51.3 | 59.8 |
| Composition of raw material solid content | Oxygen-absorbing compound C2 | % by mass | 45.7 | 45.7 | | | |
| | Oxygen-absorbing compound C3 | % by mass | | | 38.7 | 38.7 | 38.7 |
| | Oxygen-absorbing compound C4 | % by mass | | | | | |
| | Oxygen-absorbing compound C5 | % by mass | | | | | |
| | Solid content of paint 1 | % by mass | 54.3 | | 61.3 | | |
| | Solid content of paint 2 | % by mass | | 54.2 | | 61.2 | |
| | Polyol C1 | % by mass | | | | | 61.2 |
| | Polyol C2 | % by mass | | | | | |
| | Hydroxyl group-terminated polyisoprene 1 | % by mass | | | | | |
| | Biuret form of hexamethylene diisocyanate | % by mass | | | | | |
| | Oxidation promoting catalyst 1 | ppm | 251 | 252 | 258 | 258 | 258 |
| | Oxidation promoting catalyst 2 | ppm | | | | | |
| | Total | % by mass | 100.0 | 100.0 | 100.0 | 100.0 | 100.0 |
| Evaluation | Oxygen absorbed amount | cc/m² | 250 | 250 | 130 | 130 | 130 |
| | Odor sensory evaluation | - | 1 | 1 | 1 | 1 | 1 |
| | Blocking resistance evaluation | - | Good | Good | Good | Good | Good |

**[Table 10]**

| | | Unit | Example C | | | | Comparative Example C |
|---|---|---|---|---|---|---|---|
| | | | 6 | 7 | 8 | 9 | 1 |
| Formulation of coating agent composition | Oxygen-absorbing compound solution C2 | Parts by mass | 100.0 | | | | |
| | Oxygen-absorbing compound solution C3 | Parts by mass | | | | | |
| | Oxygen-absorbing compound solution C4 | Parts by mass | | 100.0 | | | |
| | Oxygen-absorbing compound solution C5 | Parts by mass | | | 250.0 | 250.0 | |
| | Paint 1 | Parts by mass | | | | | |
| | Paint 2 | Parts by mass | | | | | |
| | Polyol solution C1 | Parts by mass | | | | | |
| | Polyol solution C2 | Parts by mass | 158.0 | | | | |
| | Hydroxyl group-terminated polyisoprene 1 | Parts by mass | | | | | 100.0 |
| | Polyisocyanate solution C1 | Parts by mass | | | 25.0 | 25.0 | |
| | Oxidation promoting catalyst solution 1 | Parts by mass | 1.10 | 0.38 | 1.00 | | 0.40 |
| | Oxidation promoting catalyst solution 2 | Parts by mass | | | | 2.00 | |
| | Total | Parts by mass | 259.1 | 100.4 | 276.0 | 277.0 | 100.4 |
| | Solid content(%) | % by mass | 67.5 | 59.8 | 59.8 | 59.6 | 99.6 |
| Composition of raw material solid content | Oxygen-absorbing compound C2 | % by mass | 45.8 | | | | |
| | Oxygen-absorbing compound C3 | % by mass | | | | | |
| | Oxygen-absorbing compound C4 | % by mass | | 100.0 | | | |
| | Oxygen-absorbing compound C5 | % by mass | | | 90.9 | 90.9 | |
| | Solid content of paint 1 | % by mass | | | | | |
| | Solid content of paint 2 | % by mass | | | | | |
| | Polyol C1 | % by mass | | | | | |
| | Polyol C2 | % by mass | 54.2 | | | | |
| | Hydroxyl group-terminated polyisoprene 1 | % by mass | | | | | 100.0 |
| | Biuret form of hexamethylene diisocyanate | % by mass | | | 9.1 | 9.1 | |
| | Oxidation promoting catalyst 1 | ppm | 252 | 253 | 242 | | 160 |
| | Oxidation promoting catalyst 2 | ppm | | | | 1211 | |
| | Total | % by mass | 100.0 | 100.0 | 100.0 | 100.0 | 100.0 |
| Evaluation | Oxygen absorbed amount | cc/m² | 250 | 75 | 92 | 65 | 600 |
| | Odor sensory evaluation | - | 1 | 1 | 1 | 1 | 4 |
| | Blocking resistance evaluation | - | Good | Good | Good | Good | Poor |

### [Summary of results]

The oxygen-absorbing coating agent compositions of all the examples of the present invention exhibited an excellent balance of oxygen-absorbing properties, blocking resistance, and low odor.

On the other hand, the coating agent composition of Comparative Example C1, which was prepared with an oxygen-absorbing compound different from that of the present invention, exhibited a strong odor.

### <Evaluation method>

### [Production of test film]

The oxygen-absorbing coating agent composition was applied to PET film 1 with a bar coater such that the dry coating amount was 5.0 g/m², and then dried to provide a test film having 100 mm × 100 mm.

### [Oxygen absorbed amount]

An oxygen sensor chip (non-destructive oxygen sensor chip manufactured by Precision Sensing GmbH) and the test film produced above were enclosed inside a glass bottle with a capacity of 106 cc.

Then, the oxygen concentration was measured immediately after the enclosing and after 14 days of storage in a thermostatic chamber at 25°C, and the oxygen absorbed amount of a test film was calculated. The oxygen concentration was measured with the use of a non-destructive oxygen concentration meter (FIBOX4 OXYGEN METER) manufactured by Presence GmbH.

### [Odor sensory evaluation]

After measuring the oxygen absorbed amount, the odor of the air inside the glass bottle was evaluated sensorily according to the following evaluation criteria.

### Evaluation criteria:

0: no odor
1: faint odor
2: weak odor
3: medium odor
4: strong odor

### [Blocking resistance evaluation]

Two test films were prepared, with the coated and uncoated faces stacked together, and a load of 115 N was applied with a permanent deformation tester (constant load type) manufactured by Tester Sangyo Co., Ltd. and the film was left to stand at 40°C for one day to check whether the blocking was present or not between the two test films.

### Evaluation criteria:

Good: no blocking
Poor: blocking is present.

### REFERENCE SIGNS LIST

- 1: Laminate
- 2: Base material layer
- 3: Adhesive layer
- 4: Sealant layer
- 5: Coating layer

## Claims

1. An oxygen-absorbing compound having one or two or more unsaturated five-membered rings, wherein
any bond between five carbon atoms constituting the unsaturated five-membered ring is a carbon-carbon double bond,
a monovalent and/or divalent or higher electron donating organic group 1 is bonded to the unsaturated five-membered ring,
when there is one unsaturated five-membered ring, the five-membered ring or the organic group 1 has a functional group having active hydrogen or a group in which active hydrogen of the functional group having active hydrogen is replaced with a monovalent organic group 2, and
when there are two or more unsaturated five-membered rings, the unsaturated five-membered rings are bonded to each other via a divalent or higher organic group 2 that replaces active hydrogen of an active hydrogen group on each of the five-membered rings or the organic group 1.

2. The oxygen-absorbing compound according to claim 1, wherein a structure of the unsaturated five-membered ring or a structure composed of the unsaturated five-membered ring and the organic group 1 is derived from one or two or more selected from the group consisting of cyclopentadiene, dicyclopentadiene, norbornene, and derivatives thereof.

3. The oxygen-absorbing compound according to claim 1 or 2, wherein the organic group 2 includes a structural part derived from an isocyanate-based compound or, an isocyanate-based compound and a hydroxyl group-containing compound.

4. The oxygen-absorbing compound according to claim 3, wherein the isocyanate-based compound is one or two or more selected from the group consisting of xylylene diisocyanate, hexamethylene diisocyanate, isophorone diisocyanate, toluylene diisocyanate, diphenylmethane diisocyanate, and derivatives thereof.

5. The oxygen-absorbing compound according to claim 3 or 4, wherein the hydroxyl group-containing compound is one or two or more selected from the group consisting of polyhydric alcohols, polyolefin polyols, polyether polyols, polyester polyols, polycarbonate polyols, poly(meth)acrylic acid ester polyols, phenoxy resins, and urethane chain-extended polyols thereof.

6. The oxygen-absorbing compound according to any one of claims 1 to 5, wherein the organic group 2 has no crosslinkable functional group.

7. The oxygen-absorbing compound according to any one of claims 1 to 5, wherein
the organic group 2 has one or two or more crosslinkable functional groups, and
the crosslinkable functional group is a hydroxyl group and/or an isocyanate group.

8. The oxygen-absorbing compound according to any one of claims 1 to 5, wherein the oxygen-absorbing compound contains one or two or more selected from the group consisting of compounds represented by the following formulas (1) to (4); 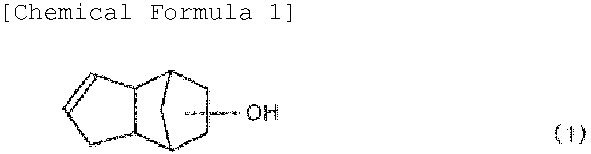 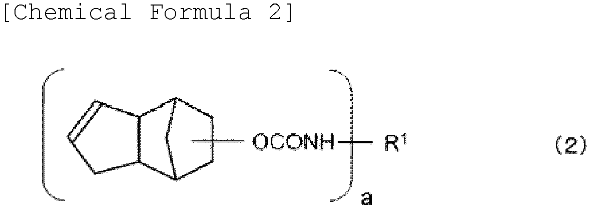 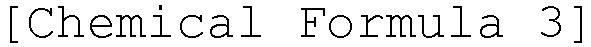 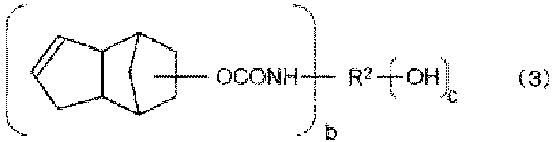 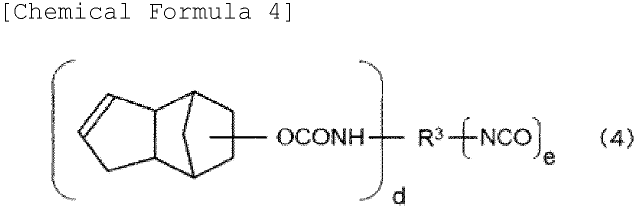 wherein a to e each represent a number of 1 or more, and each of R¹, R², and R³ represents an organic group having 1 or more carbon atoms, includes at least an alkylene and/or phenylene structure, and optionally further includes a structure derived from one or two or more selected from the group consisting of polyhydric alcohols, polyolefin polyols, polyether polyols, polyester polyols, polycarbonate polyols, poly(meth)acrylic acid ester polyols, phenoxy resins, and urethane chain-extended polyols thereof.

9. The oxygen-absorbing compound according to any one of claims 1 to 5, wherein the oxygen-absorbing compound contains a compound represented by the following formula (5) : 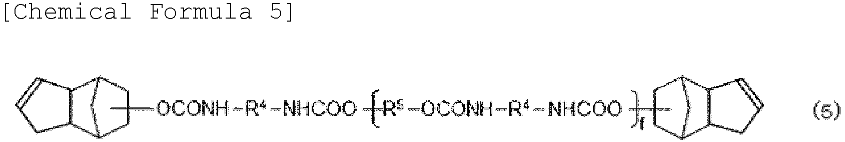 wherein f represents a number of 0 or more, and each of R⁴ and R⁵ represents an organic group having 1 or more carbon atoms, represents the organic group including at least an alkylene and/or phenylene structure, and optionally further includes a structure derived from one or two or more selected from the group consisting of polyhydric alcohols, polyolefin polyols, polyether polyols, polyester polyols, polycarbonate polyols, poly(meth)acrylic acid ester polyols, phenoxy resins, and urethane chain-extended polyols thereof.

10. An oxygen-absorbing composition comprising the oxygen-absorbing compound according to any one of claims 1 to 9 and an oxidation promoting catalyst.

11. An oxygen-absorbing adhesive composition comprising at least the oxygen-absorbing compound according to any one of claims 1 to 9 and an oxidation promoting catalyst.

12. The oxygen-absorbing adhesive composition according to claim 11, wherein the oxidation promoting catalyst is a peroxide or a compound including a cation composed of a transition metal.

13. The oxygen-absorbing adhesive composition according to claim 12, wherein the compound including a cation composed of a transition metal is a metal soap composed of: a transition metal compound capable of releasing a cation or complex, composed of a transition metal; and an anion or ligand composed of a fatty acid.

14. The oxygen-absorbing adhesive composition according to any one of claims 11 to 13, wherein
the oxygen-absorbing adhesive composition further contains a modifier, and
the modifier contains an isocyanate-based compound and/or a hydroxyl group-containing compound.

15. The oxygen-absorbing adhesive composition according to claim 14, wherein the isocyanate-based compound is one or two or more selected from the group consisting of xylylene diisocyanate, hexamethylene diisocyanate, isophorone diisocyanate, toluylene diisocyanate, diphenylmethane diisocyanate, and derivatives thereof.

16. The oxygen-absorbing adhesive composition according to claim 14 or 15, wherein the hydroxyl group-containing compound includes one or two or more selected from the group consisting of polyester polyols, poly(meth)acrylic acid ester polyols, polyalkylene ether diols, and urethane chain-extended polyols thereof.

17. An oxygen-absorbing coating agent composition comprising at least the oxygen-absorbing compound according to any one of claims 1 to 9 and an oxidation promoting catalyst.

18. The oxygen-absorbing coating agent composition according to claim 17, wherein the oxidation promoting catalyst is a peroxide or a compound including a cation composed of a transition metal.

19. The oxygen-absorbing coating agent composition according to claim 18, wherein the compound including a cation composed of a transition metal is a metal soap composed of: a transition metal compound capable of releasing a cation or complex, composed of a transition metal; and an anion or ligand composed of a fatty acid.

20. The oxygen-absorbing coating agent composition according to any one of claims 17 to 19, further comprising a modifier, wherein the modifier contains an isocyanate-based compound and/or a hydroxyl group-containing compound.

21. The oxygen-absorbing coating agent composition according to claim 20, wherein the isocyanate-based compound is one or two or more selected from the group consisting of xylylene diisocyanate, hexamethylene diisocyanate, isophorone diisocyanate, toluylene diisocyanate, diphenylmethane diisocyanate, and derivatives thereof.

22. The oxygen-absorbing coating agent composition according to claim 20 or 21, wherein the hydroxyl group-containing compound includes one or two or more selected from the group consisting of polyester polyols, poly(meth)acrylic acid ester polyols, polyalkylene ether diols, and urethane chain-extended polyols thereof, vinyl chloride-vinyl acetate copolymers, cellulose acetate-based resins, styrene-maleic acid ester-based copolymers, polyamide-based resins, and polyimide-based resins.
